(19) Europäisches Patentamt
European Patent Office
Office européen des brevets

(11) Numéro de publication: **0 105 006**
**B1**

(12) # FASCICULE DE BREVET EUROPEEN

(45) Date de publication du fascicule du brevet: **19.03.86**

(21) Numéro de dépôt: **83401886.3**

(22) Date de dépôt: **27.09.83**

(51) Int. Cl.⁴: **C 07 C 121/75**, C 07 C 61/16, C 07 C 69/608, C 07 C 69/743, C 07 D 213/64, C 07 D 233/74, C 07 D 307/42, A 01 N 53/00, A 23 L 1/16 // C07C62/34, C07C69/757

(54) **Cyclopropane carboxylates à structure allénique, leur procédé et leurs intermédiaires de préparation, leur application à la lutte contre les parasites et les compositions les renfermant.**

(30) Priorité: **29.09.82 FR 8216372**

(43) Date de publication de la demande: **04.04.84 Bulletin 84/14**

(45) Mention de la délivrance du brevet: **19.03.86 Bulletin 86/12**

(84) Etats contractants désignés: **CH DE FR GB IT LI NL**

(56) Documents cités:
**EP - A - 0 007 255**
**EP - A - 0 050 093**

(73) Titulaire: **ROUSSEL-UCLAF, 35, boulevard des Invalides, F-75007 Paris (FR)**

(72) Inventeur: **Tessier, Jean, 30, rue Jean Moulin, F-94300-Vincennes (FR)**
Inventeur: **Demoute, Jean-Pierre, 249bis, rue de Rosny, F-93100-Montreuil-sous-Bois (FR)**
Inventeur: **Cadiergue, Joseph, 6, rue Jules Princet, F-93600-Aulnay-sous Bois (FR)**

(74) Mandataire: **Fritel, Hubert et al, ROUSSEL-UCLAF 111, route de Noisy Boîte Postale no. 9, F-93230 Romainville (FR)**

**EP 0 105 006 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1986

**Description**

L'invention concerne des cyclopropane carboxylates à structure allénique, leur procédé et leurs intermédiaires de préparation, leur application à la lutte contre les parasites et les compositions les renfermant.

L'invention a pour objet sous toutes les formes isomères possibles, les composés de formule générale I

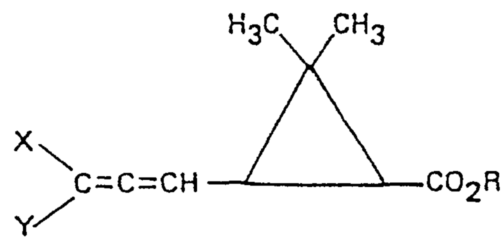

dans laquelle R represente le reste d'un alcool utilisé dans la synthèse des pyrethrinoïdes et

ou bien X at Y raprésentent chacun un atome d'hydrogène,

ou bien Y représente un atome d'hydrogène et X un atome de chlore ou de brome,

ou bien X représente un atome d'hydrogène ou d'halogène ou un radical alcoyle renfermant de 1 à 18 atomes de carbone et Y représente un radical $CO_2R'$, R' représentant un radical alcoyle renfermant de 1 à 18 atomes de carbone.

par atome d'halogène, on entand de préférence un atome de chlore, de brome ou de fluor.

Lorsque X represente un radical alcoyle, il s'agit de preférence d'un radical méthyle, éthyle, n-propyle, n-butyle, n-pentyle, isopropyle, isobutyle ou terbutyle.

Comme valeurs preférées du radical R', on peut citer les valeurs préferées indiquées pour X.

L'invention a notamment pour objet les composés de formule (I) pour lesquels X et Y représentent chacun un atome d'hydrogène.

L'invention a tout particulièrement pour objet les composés de formule I pour lesquels R représente:

- soit un radical alcoyle renfermant de 1 à 18 atomes de carbone,
- soit un radical benzyle éventuellement substitué par un ou plusieurs radicaux, choisis dans le groupe constitué par les radicaux alcoyles

comportant de 1 à 4 atomes de carbone, les radicaux alcényles
comportant de 2 à 6 atomes de carbone, les radicaux alcényloxy
comportant de 2 à 6 atomes de carbone, les radicaux alcadiényles
comportant de 4 à 8 atomes de carbone, le radical méthylène dioxy et les atomes d'halogène,

- soit un groupement

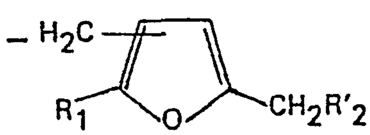

dans lequel le substituant $R_1$ représente un atome d'hydrogène ou un radical méthyle et le substituant $R'_2$ un radical aryle monocyclique ou un groupement -C≡CH et notamment un groupement 5-benzyl 3-furyl méthyle,

- soit un groupement

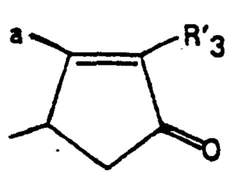

dans lequel a représente un atome d'hydrogène ou un radical methyle et $R_3$ représente un radical organique aliphatique comportant 2 à 6 atomes de carbone et une ou plusieurs insaturations carbone-carbone et

notamment le radical $-CH_2-CH=CH_2$, $-CR_2=CH=CH-CE_3$ $-CR_2-CH=CH-CH=CH_2$, $-CH_2-CR=CH-CH_2-CH_3$,$-CH_2-C\equiv CH$

- soit un groupement

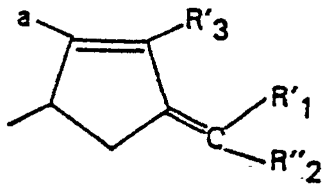

dans lequel a et R'$_3$ conservent la même signification que précédemment, R'$_1$ et R''$_2$ identiques ou différents, représentent un atome d'hydrogène, un atome d'halogene, un radical alcoyle renfermant de 1 à 6 atomes de carbone, un radical aryle comportant de 6 à 10 atomes de carbone, un groupement alcoyloxycarbonyle comportant de 2 à 5 atomes de carbone, ou un groupement cyano,

-soit un groupement

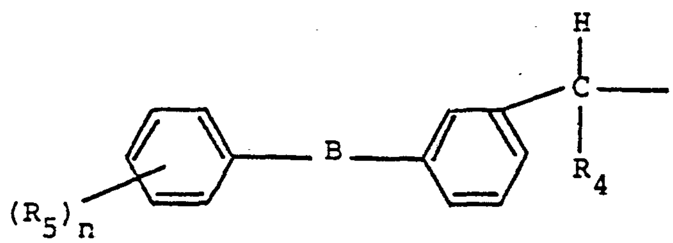

dans lequel B représente un atome d'oxygène ou de soufre, un groupement

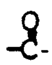 ou $-CH_2-$ et $R_4$ représente un atome d'hydrogène,un radical-C≡ N ou un radical méthyle,un radioal $-CO-NH_2$, un radical $-CSNH_2$ ou un radical $-C\equiv CH$, $R_5$ représente un atome d'halogène ou un radical méthyle etn représente un nombre egal a 0, 1 ou 2, et notamment le groupement 3-phénoxy benzyle, α-cyano 3-phénoxy tenzyle,α -éthynyl 3-phénoxy tenzyle, 3-benzoyl tenzyle, 1-(3-phénoxyphényl) éthyle ou α-thioamido 3-phénoxy benzyle,

-soit un groupement

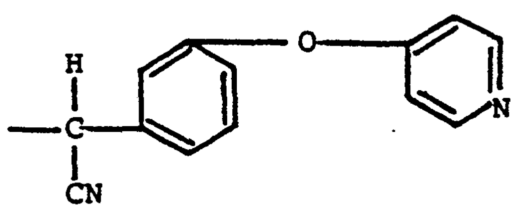

- soit un groupement

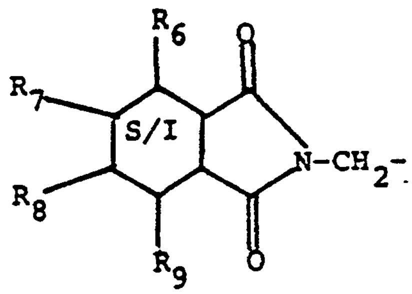

dans lequel les substituants $R_6$, $R_7$, $R_8$, $R_9$ représentent un atome d'hydrogène, un atome de chlore ou un radical méthyle et dans lequel S/I symbolise un cycle aromatique ou un cycle analogue dihydro, tétrahydro ou hexahydro,

-soit un groupement (succinimido ou maléimido)-$CH_2$-,

- soit un groupement

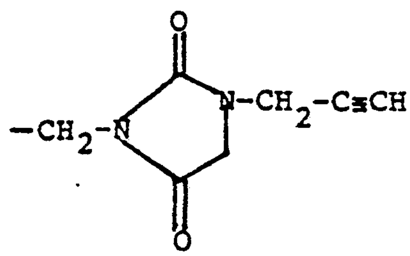

- soit un groupement

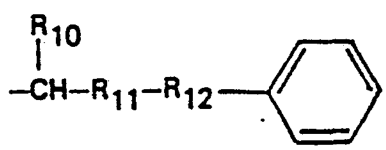

dans lequel $R_{10}$ représente un atome d'hydrogène ou un radical CN, $R_{12}$ repréêente un radical -$CH_2$- ou un atome d'oxygène, $R_{11}$ représente un radical thiazolyle ou thiadiazolyle dont la liaison avec

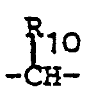

peut se trouver à l'une quelconque des positions disponibles, $R_{12}$ étant lié à $R_{11}$ par l'atome de carbone compris entre l'atome de soufre et un atome d'azote,

- soit un groupement

- soit un groupement

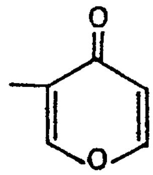

- soit un groupement

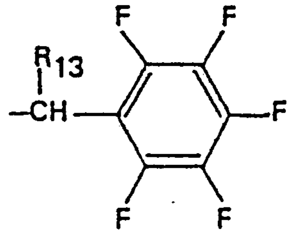

dans lequel $R_{13}$ représente un atome d'hydrogène ou un radical CN,
- soit un groupement

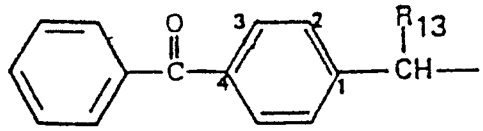

dans lequel $R_{13}$ est defini comme ci-dessus,
- soit un groupement

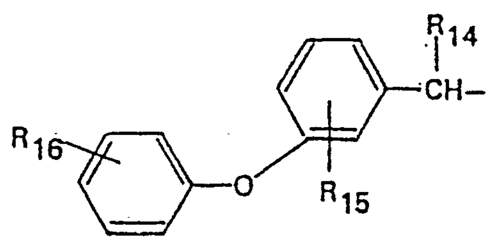

dans lequel $R_{14}$ représente un atome d'hydrogène, un radical methyle, éthynyle ou cyano et $R_{15}$ et $R_{16}$, identiques ou différents, représentent un atome-d'hydrogène, de fluor ou de brome,
- soit un groupement

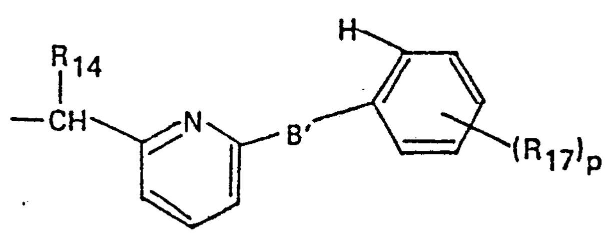

dans lequel $R_{14}$ est défini comme ci-dessus, chacun des $R_{17}$ représente indépendamment un groupement alcoyle renfermant de 1 à 4 atomes de carbone, alcoxy renfermant de 1 à 4 atomes de carbone, alcoylthio renfermant de 1 à 4 atomes de carbone, alcoyl sulfonyl renfermant de 1 à 4 atomes de carbone, trifluorométhyl, 3,4-méthylène dioxy, chloro, fluoro ou bromo, p représente un nombre égal à 0, 1 ou 2 et B' représente un atome d'oxygène ou un atome de soufre.

Lorsque R représente un radical alcoyle, il s'agit de préférence du radical méthyle, éthyle, n-propyle, n-butyle,

n-pentyle, isopropyle,isobutyle ou terbutyle.

Lorsque R représente un radical benzyle substitué par un ou plusieurs radicaux alcoyle, il s'agit de préférence du radical méthyle ou éthyle.

Lorsque R représente un radical benzyle substitué par un ou plusieurs radicaux alcényle, il s'agit de préférence de radicaux vinyle, allyle, 2-méthylallyle ou isobutényle.

Lorsque R représente un radical benzyle substitué par un radical alcadiényle, il s'agit de préférence d'un radical benzyle substitué par un radical alcadiényle renfermant 4, 5 ou 6 atomes de carbone.

Lorsque R représente un radical benzyle substitué par un ou plusieurs alcényloxy, il s'agit de préférence de radicaux vinyloxy, allyloxy, 2-méthylallyloxy ou isobuténylоxy.

Lorsque R représente un radical benzyle substitué par un ou plusieurs atomes d'halogène, il s'agit de préférence d'atomes de chlore, de brome ou de fluor.

Lorsque R représente le radical:

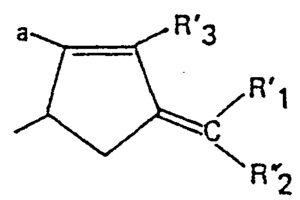

$R_3'$ représente de préférence le radical $-CH_2-CH=CH_2$, $-CH_2-CH=CH-CH_3$, $-CH_2-CH=CH-CH=CH_2$ ou $-CH_2-CH=CH-CH_2-CH_3$.

Lorsque $R'_1$ et (ou) $R''_2$ représentent un atome d'halogène il s'agit de préférence d'un atome de chlore, de brome ou de fluor.

Lorsque $R_1'$ et (ou) $R_2''$ représentent un radical alcoyle, il s'agit de préférence du radical méthyle, éthyle, n-propyle ou n-butyle.

Lorsque $R_1'$ et (ou) $R''_2$ représentent un radical aryle, il s'agit de préférence du radical phényle.

Lorsque $R_1'$ et (ou) $R''_2$ représentent un radical alcoyloxy carbonyl; il s'agit de préférence du radical méthoxy carbonyl ou éthoxycarbonyl.

Lorsque dans le radical:

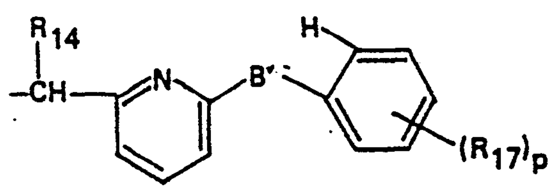

$R_{17}$ représente un radical alcoyle, alcoxy, alcoylthio ou alcoylsulfonyl, il s'agit de préférence du radical méthyle, éthyle, méthoxy, éthoxy, méthylthio, éthylthio, méthyl sulfonyl ou éthyl sulfonyl.

L'invention a notamment pour objet les composés,caractérisés en ce que le groupement R représente

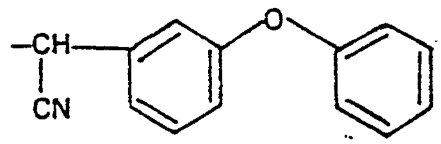

ceux caractérisés en ce que le groupement R représente

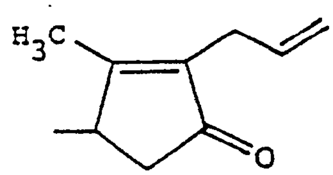

ceux caractérisés en ce que le groupement R représente

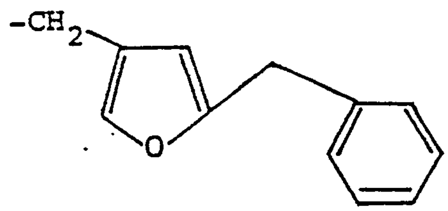

ainsi que ceux caractérisés en ce que le groupement R représente

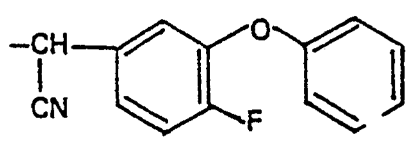

L'invention a également pour objet un procédé de préparation des composés de formule générale I, caractérisé en ce que l'on fait réagir un acide de formule générale (II)

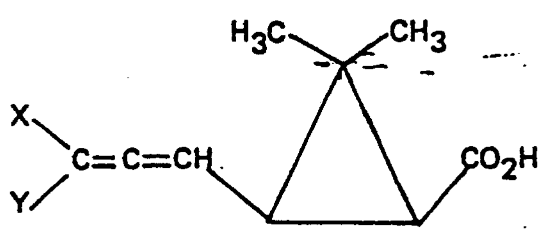

II

formule dans laquelle X et Y conservent les significations précédentes ouun derive fonctionnel de cet acide, ave un alcool R-OH, R conservant la même signification que précédemment ou avec un dérive fonctionnel de cet aloool.

Selon un mode préféré du procédé de l'invention, l'estérification de l'acide par l'alcool est effectuée en présence de dicyclohexylcarbodiimide et de 4-diméthyl aminopyridine. L'invention a plus précisément pour objet un procédé de préparation des composés de formule générale (I) dans lesquels X=Y=H, caractérisé en ce que l'on prepare l'acide IR,trans ou 1R,cis 2,2-diméthyl 3-(1,2-propadiényl) cyclopropane carboxylique (produit de formule (II) dans lequel X=Y=H) en faisant réagir le butyllithium sur un ester d'alcoyle de l'acide IR,trans ou 1R,cis 2,2-diméthyl 3-(2,2-dibromovinyl) cyclopropane carboxylique, alcoyle représentant un alcoyle de 1 à 8 atomes de carbone, puis en faisant réagir le 1R, trans ou IR, cis 2,2-diméthyl 3-ethynyl cyclopropane carboxylate d'alcoyle résultant avec de la diisopropylamine, du formaldéhyde et du bromure cuivreux, puis en soumettant le IR, trans ou le 1R, cis 2,2-diméthyl 3-(1,2-propadiényl) cyclopropane carboxylate

d'alcoyle formé, à l'action d'un agent basique en présence d'eau.

Les composés de formule générale I pour lesquels Y=H et X=Cl ou Br peuvent être préparés de la manière illustrée par le schéma suivant:

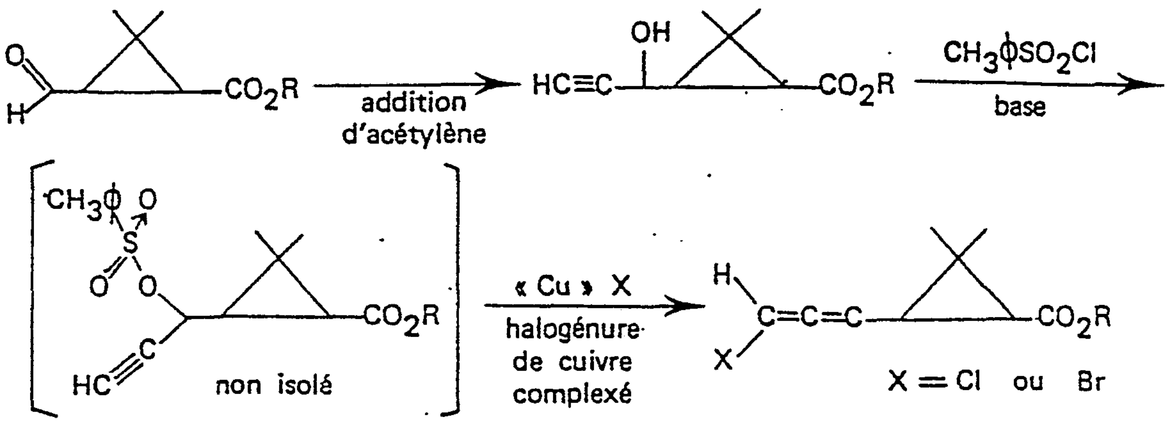


Les composés de formule générale I pour lesquels X=H ou halogène ou alcoyle comportant de 1 à 18 atomes de carbone et $Y=CO_2R'$, R' étant un radical alcoyle renfermant jusqu'è 18 atomes de carbone peuvent être préparés de la manière illustrée par le schéma suivant:

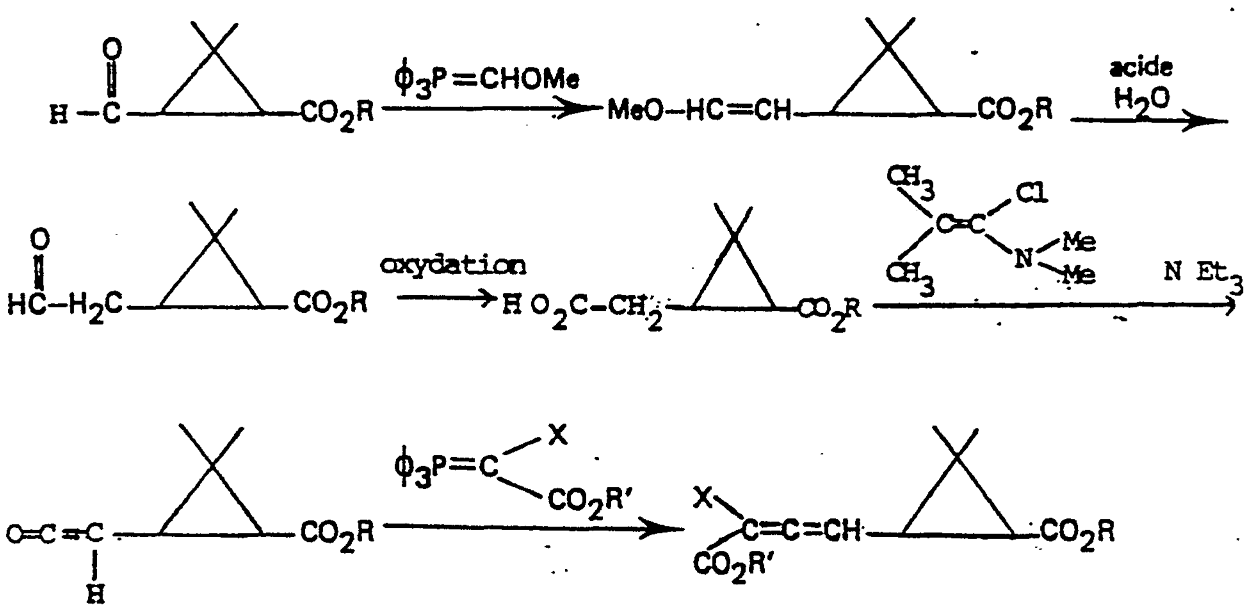


Différentes illustrations de procédés de préparation des composés de formule (I) sont fournies ci-après dans la partie expérimentale.

Les composés de formule (1) présentent d'intéressantes propriétés qui permettent leur utilisation dans la lutte contre les parasites. Il peut s'agir par exemple de la lutte contre les parasites des végétaux, les parasites des locaux et les parasites des animaux à sang chaud. C'est ainsi que l'on peut utiliser les produits de l'invention pour lutter contre les insectes, les nématodes et les acariens parasites des végétaux et des animaux.

L'invention a donc aussi pour objet l'application des composes de formule (I) à la lutte contre les parasites des végétaux, les parasites des locaux et les parasites des animaux à sang chaud.

L'invention a egalement pour objet les compositions destinées à la lutte contre les parasites des végétaux, les parasites des locaux et les parasites des animaux à sang chaud, caractérisés en ce qu'elles renferment comme principe actif au moins un des produits définis précédemment.

Les produits de formule (I) peuvent être utilisés notamment pour lutter contre les insectes dans le domaine agricole, pour lutter par exemple contre les pucerons, les larves de lépidoptères et les coléoptères. Ils sont utilisés à des doses comprises entre 10 g et 300g de matière active à l'hectare.

Les produits de formule (I) peuvent également être utilisés pour lutter contre les insectes dans les locaux,

pour lutter notamment contre les mouches, les moustiques et les blattes.

L'invention a donc notamment pour objet les compositigns insect ici des renfermant comme principe actif au moins l'un des produits définis précédemment.

Parmi les compositions notamment insecticides préférées de l'invention, on peut citer tout spécialement les compositions renfermant les composés décrits dans les exemples.

Les compositions insecticides selon l'invention contiennent de préférence de 0,005% à 10% en poids de matière active.

Selon un mode opératoire avantageux, pour un usage dans les locaux, les compositions, selon l'invention, sont utilisées sous forme de compositions fumigantes.

Les compositions selon l'invention peuvent alors être avantageusement constituées, pour la partie non active, d'un serpentin combustible, ou encore d'un substrat fibreux incombustible. Dans ce dernier cas, le fumigant obtenu après incorporation de la matière active est placé sur un appareil chauffant tel qu'un électro émanateur.

Dans le cas où l'on utilise un serpentin insecticide, le support inerte peut être par exemple, composé de marc de pyrèthre, poudre de Tabu (ou poudre de feuilles Machilus Thumbergii), poudre de tige de pyrèthre, poudre de feuille de cèdre, poudre de bois (telle que de la sciure de pin), amidon et poudre de coque de noix de coco.

La dose de matière active peut alors être, par exemple, de 0,03 à 1% en poids.

Dans le cas où l'on utilise un support fibreux incombustible, la dose de matière active peut alors être par exemple, de 0,03 à 95% en poids.

Les compositions selon l'invention pour un usage dans les locaux peuvent aussi être obtenues en préparant une huile pulvérisable à base de principe actif, cette huile imbibant la mèche d'un lampe et étant alors soumise à la combustion.

La concentration du principe actif incorporé à l'huile est, de préférence, de 0,03 à 95% en poids.

Les produits de formule (I) peuvent aussi être utilisés pour lutter contre les acariens et les nématodes parasites des végétaux. L'invention a donc pour objet les compositions acaricides renfermant comme principe actif au moins l'un des produits définis précédemment.

L'invention a encore pour objet les compositions nématicides renfermant comme principe actif au moins l'un des produits définis précédemment.

Les compositions acaricides et nématicides peuvent se présenter notamment sous forme de poudres, granulés, suspensions, émulsions, solutions.

Pour l'usage acaricide, on utilise de préférence des poudres mouillables, pour pulvérisation foliaire, contenant de 1 à 80% en poids de principe actif, ou des liquides pour pulvérisation foliaire contenant de 1 à 500 g/l de principe actif. On peut egalement employer des poudres pour poudrage foliaire contenant de 0,05 à 3% en poids de matière active.

Pour l'usage nematicide, on utilise de préférence des liquides pour traitement des sols contenant de 300 à 500 g/l de principe actif.

Les composés acaricides et nématicides selon l'invention sont utilisés de préférence, à des doses comprises entre 1 et 100 g de matière active à l'hectare.

Ces compositions insecticides, acaricides et nématicides sont préparées selon les procédés usuels de l'industrie agrochimique.

Elles peuvent être additionnées éventuellement d'un ou plusieurs autres agents pesticides. Ces compositions peuvent se présenter sous forme de poudres, granulés, suspensions, émulsions, solutions, solutions pour aérosols, bandes combustibles, appâts ou autres préparations employées classiquement pour l'utilisation de ce genre de composés.

Outre le principe actif, ces compositions contiennent, en général un véhicule et/ou un agent tensio-actif, non ionique, assurant, en outre, une dispersion uniforme des substances constitutives du mélange. Le véhicule utilisé peut être un liquide, tel que l'eau, l'alcool ou les hydrocarbures ou autres solvants organi-ques, une huile minérale, animale ou végétale, une poudre telle que le talc, les argiles, les silicates, le kieselguhr ou un solide combustible.

Les produits de formule (I) sont de plus photostables et ne sont pas toxiques pour les mammifères.

L'ensemble de ces propriétés fait des produits de formule (I) des produits qui correspondent parfaitement aux exi-gences de l'industrie agrochimique moderne: ils permettent de protéger les récoltes tout en préservant l'environnement.

Les composés de formule (I) peuvent encore être utilisés pour lutter contre les acariens parasites des animaux, pour lutter par exemple contre les tiques et nnotamment les tiques de l'espèce Boophilus, ceux de l'espèce Hyalomnia, ceux de l'espèce Amblyomnia et ceux de l'espèce Rhipicephalus ou pour lutter contre toutes sortes de gales et notamment la gale sarcoptique, la gale psoroptique et la gale chorioptique. Ils peuvent aussi être utiles contre les poux et les helminthes.

L'invention a donc pour objet les compositions caractérisées en ce qu'elles sont utilisées dans la lutte contre les parasites des animaux à sang chaud, notamment contre les tiques et les gales.

Lorsqu'il s'agit de lutter contre les parasites des animaux, les composés de l'invention peuvent être administrés par voie externe sous forme de vaporisation, bain, badigeonnage, poudrage.

Ils peuvent être également administrés par badigeonnage de l'épine dorsale selon la méthode dite méthode "Pour on".

Lorsqu'il s'agit de lutter contre les acariens parasites des animaux, on peut incorporer très souvent les

produits de l'invention dans des compositions alimentaires en association avec un mélange nutritif adapté à l'alimentation animale. Le mélange nutritiel peut varier selon l'espèce animale; il peut renfermer des céréales, des sucres et des grains, des tourteaux de soja, d'arachide et de tournesol, des farines d'origine animale, par exemple des farines de poissons, des acides aminés de synthèse, des sels minéraux, des vitamines et des antioxydants.

L'invention a également pour objet les compositions destinées à l'alimentation animale renfermant comme principe actif au moins l'un des produits définis précédemment.

L'invention a aussi pour objet les associations douées d'activité insecticide, acaricide ou nématicide, caractérisées en ce qu'elles contiennent comme matière active, d'une part un au moins des composés de formule générale (I) et d'autre part, un au moins des esters pyréthrinoïdes choisis dans le groupe constitué par les esters d'alléthrolones, d'alcool 3, 4, 5, 6-tétrahydrophtalimido méthylique, d'alcool 5-benzyl 3-furyl méthylique, d'alcool 3-phénoxy benzylique et d'alcools α-cyano 3-phénoxy benzyliques des acides chrysanthémiques, par les esters d'alcool 5-benzyl 3-furyl méthylique des acides 2,2-diméthyl 3-(2-oxo 3-tétrahydrothiophénylidène methyl) cyclopropane-1-carboxyliques, par les esters d'alcool 3-phénoxy benzylique et d'alcools α-cyano-3-phénoxy benzyliques des acides 2,2-diméthyl 3-(2,2-dichlorovinyl) cyclopropane-1-carboxyliques, par les esters d'alcools-α-cyano. 3-phénoxy benzyliques d'acides 2,2-diméthyl 3-(2,2 dibromovinyl) cyclopropane-1-carboxyliques, par les esters d'alcool 3-phénoxy benzylique des acides 2-parachlorophenyl isopropyl acétiques, par les esters d'alléthrolones, d'alcool 3, 4, 5, 6-tétrahydro-phtalimido méthylique, d'alcool 5-benzyl 3-furyl méthylique, d'alcool 3-phenoxy benzylique et d'alcools α-cyano 3-phénoxy benzyliques des acides 2,2-diméthyl 3-(1,2-2,2-tétrahaloéthyl) cyclopropane-1-carboxyliques, dans lesquels "halo" représente un atome de fluor, de chlore ou de brome, étant entendu que les copules acides et alcools des esters pyrethrinoides ci-dessus, peuvent exister sous toutes leurs formes stéréoisomères possibles.

L'invention a aussi pour objet les compositions insecticides, acaricides ou nématicides décrites ci-dessus caractérisées en ce qu'elles renferment en outre un synergiste des pyréthrinoides. Les synergistes que l'on peut utiliser sont des produits bien connus de l'homme de métier.

Les composés de formule (II) utilisés pour préparer les produits de formule (I) sont des produits nouveaux et sont en eux-mêmes un des objets de l'invention.

L'invention à donc encore pour objet les produits de formule (II) à titre de produits chimiques nouveaux et notamment à titre d'intermédiaires nécessaires à la préparation des composes de formule (I).

L'invention a plus particulièrement pour objet les composés de formule (II) pour lesquels X=Y=H.

Les exemples suivants illustrent l'invention sans toutefois la limiter.

**EXEMPLE 1: 1R ci-s 2,2-diméthyl 3-(1,2-propadienyl) cyclopropane carboxylate de RS cyano (3-phenoxyphényl) méthyle.**

Dans une solution de 1,52 g d'acide 1R cis 2,2-diméthyl 3-(1,2-propadiényl) cyclopropane carboxylique dans 30 $cm^3$ de chlorure de methylène, on introduit à 0°C 2,06 g de dicyclohexylcarbodiimide, quelques cristaux de 4-diméthylaminopyridine, agite pendant 15 minutes à 0°C, introduit une solution de 2,47 g d'alcool RS cyano (3-phénoxyphényl) méthylique dans 10 $cm^3$ de chlorure de méthylène, agite pendant 4 heures à 20°C, filtre, concentre è sec par distillation sous pression réduite, chromatographie le résidu sur silice en éluant par un mélange d'éther de pétrole éb. 35°-70°C et de benzène (7/3) puis par un mélange de benzène et d'ether de pétrole éb. 35°-70°C (7/3) et obtient 2,89 g de IR cis, 2,2-diméthyl 3-(1,2-propadiényl) cyclopropane carboxylate de RS-cyano (3-phénoxyphényl) méthyle.

**Analyse:** $C_{23}H_{21}NO_3$ (359,428)

| | C% | H% | N% |
|---|---|---|---|
| Calculés : | 76,86 | 5,89 | 3,90 |
| Trouvés : | 76,60 | 5,90 | 3,80 |

**Spectre U.V** (ethanol)

Inflexion à 230 nm ($E^{1\%}_{1cm}$: 342) ; inflexion à 269 nm ($E^{1\%}_{1cm}$: 49) ;
Inflexion à 279 nm ($E^{1\%}_{1cm}$ : 53) ; maximum à 278 nm ($E^{1\%}_{1cm}$ : 58).

**EXEMPLE 2: 1R cis 2,2-diméthyl 3-(1,2-propadiényl) cyclopropane carboxylate de 5-benzyl 3-furyl méthyle**

Dans une solution de 1,52 g d'acide 1R cis 2,2-diméthyl 3-(1,2-propadiényl) cyclopropane carboxylique dans 40 $cm^3$ de chlorure de méthylène, on introduit à 0°C, 4,06 g de dicyclohexylcarbodiimide, quelques cristaux de 4-dimethylaminopyridine, agite pendant 15 minutes à 0°C, introduit à 0°C une solution de 2,07 g de 5-benzyl 3-furyl méthanol dans 10 $cm^3$ de chlorure de methylène, agite pendant 4 heures à 20°C, filtre, concentre à sec par distillation sous pression réduite, chromatographie le résidu sur silice en éluant par un mélange de benzène et d'éther de pétrole eb. 35°-70°C (1/1) et obtient 2,72 g de IR cis 2,2-diméthyl3-(1,2-propadiényl) cyclopropane carboxylate de 5-benzyl 3-furyl méthyle. Analyse: $C_{21}H_{22}O_3$ (322,407)

| | C% | H% |
|---|---|---|
| Calculés | 78,23 | 6,88 |
| Trouvés | 77,90 | 6,70 |

**Spectre U.V.** (éthanol)

Inflexion 251 nm ($E^{1\%}_{1cm}$ : 522) ; inflexion à 252 nm ($E^{1\%}_{1cm}$ : 10 ) ; maximum à 258 nm ($E^{1\%}_{1cm}$ : 9) ; maximum à 268 nm ($E^{1\%}_{1cm}$ : 6)

**EXEMPLE 3: 1R trans 2,2-dimethyl 3-(1 2-propadiényl) cyclopropane carboxylate de 5-benzyl 3-furyl méthyle**

Dans une solution de 900 mg d'acide IR trans 2,2-diméthyl 3-(1,2-propadiényl) cyclopropane carboxylique dans 20 $cm^3$ de chlorure de méthylène on introduit à 0°C 1,22 g de dicyclohexylcarbodiimide, quelques cristaux de 4-diméthylaminopyridine, agite pendant 15 minutes à 0°C, introduit 1,23 g de 5-benzyl 3-furyl methanol en solution dans 7 $cm^3$ de chlorure de méthylène, agite pendant 24 heures à 20°C, filtre, concentre à sec par distillation sous pression réduite, chromatographie le residu sur silice en-éluant par un mélange d'ether de petrole (éb.35°-70°C) et de benzène (3/7) puis par un mélange d'éther de pétrole éb. 35°-70°C benzène (7/3) et obtient 825 mg de1R trans 2,2 diméthyl 3-(1,2-propadiényl) cyclopropane carboxylate de 5-benzyl 3-furyl méthyle $[\alpha]_D = -24°$ (C=0,6% chloroforme).

**Analyse:** $C_{21}H_{22}O_3$ (322,407)

| | C% | H% |
|---|---|---|
| Calculés : | 78,23 | 6,88 |
| Trouvés : | 78,0 | 6,9 |

**Spectre U.V.** (ethanol)

Inflexion 295 nm ($E^{1\%}_{1cm}$ : 489) ; maximum 251 nm ($E^{1\%}_{1cm}$ : 8,5) ; maximum 259 nm ($E^{1\%}_{1cm}$ : 8,4) ; maximum 268 ($E^{1\%}_{1cm}$ : 5,1) ;

**EXEMPLE 4: 1R cis 2,2-dimethyl 3-(1,2-propadienyl) cyclopropane carboxylate de S 3-allyl 2-méthyl 4-oxo 2-cyclopenten-1-yle**

Dans une solution de 10 g d'acide IR cis 2,2-diméthyl 3-(1,2-propadiényl) cyclopropane carboxylique dans 200 $cm^3$ de chlorure de methylène, on introduit à 0°C, 13,6 g de dicyclohexylcarbodiimide et 100 mg de 4-diméthylaminopyridine, agite pendant 25 minutes à 0°C, ajoute une solution de 11 g de S 3-allyl 2-méthoxy 1-hydroxy 2-cyclopenten-4-one dans 35 $cm^3$ de chlorure de méthylène, agite pendant 5 heures à 20°C, filtre, concentre à sec par distillation sous pression réduite, ajoute au résidu 25 $cm^3$ d'hexane, refroidit à 0°C isole par essorage l'insoluble formé, concentre le filtrat à sec par distillation sous pression réduite, chromatographie le residu sur gel de silice en éluant par un mélange d'hexane et d'acétate d'ethyle (9/1) et obtient 16,8 g de 1R cis 2,2-diméthyl 3-(1,2-propadiényl) cyclopropane carboxylate de S 2-méthyl 3-allyl 4-oxo 2-cyclopenten-1-yle. $[\alpha]_D = +46°$ (C=1% chloroforme).

**Analyse:** $C_{18}H_{22}O_3$ (286,37)

| | C% | H% |
|---|---|---|
| Calculés : | 75,50 | 7,74 |
| Trouvés : | 75,6 | 7,9 |

**EXEMPLE 5: 1R trans 2,2-diméthyl 3-(1,2-propadiényl) cyclopropane-1-carboxylate de S 2-méthyl 3-allyl 4-oxo 2-cyclopenten-1-yle**

Dans une solution de 900 mg d'acide IR trans 2-2-diméthyl 3-(1,2-propadienyl) cyclopropane-1-carboxylique dans 20 $cm^3$ de chlorure de méthylène, on introduit à 0°C, 1,22 g de dicyclohexylcarbodiimide, quelques cristaux de 4-diméthylaminopyridine, agite pendant 15 minutes à 0°C, introduit une solution de 990 mg de S-3-allyl 2-méthyl 1-hydroxy 2-cyclopenten-4-one dans 7 $cm^3$ de chlorure de méthylène, agite pendant 24 heures à 20°C, filtre, concentre à sec par distillation sous pression réduite, chromatographie le résidu sur gel de silice en éluant avec un mélange de benzène et d'acétate d'éthyle (97/O3) et obtient 468 mg de IR trans 2,2-dime-thyl 3-(1,2-propadiényl) cyclopropane 1-carboxylate de S 3-allyl 2-méthyl 4-oxo 2-cyclopentène-1-yle $[\alpha]_D$=-26°(C=0,5% chloroforme) Dichroïsme circulaire (ethanol)

maximum à 230 nm (Δε:-22,9); maximum à 315 nm (Δε:+2,70)

**Spectre U.V.** - (éthanol)

maximum à 229 nm ($E^{1\%}_{1cm}$ : 567)

inflexion à 280 nm ($E^{1\%}_{1cm}$ : 7) ; inflexion à 295 nm ($E^{1\%}_{1cm}$ : 6)

**Microanalyse:**

| | C% | H% |
|---|---|---|
| Calculés : | 75,50 | 7,74 |
| Trouvés : | 75,50 | 7,80 |

**EXEMPLE 6: 1R trans 2,2-diméthyl 3=(1,2-propadiényl) cyclcpropane 1-carboxylate de S cyano (3-phénoxyphényle) méthyle**

Dans une solution de 900 mg d'acide 1R trans 2,2-diméthyl 3-(1,2-propadiényl) cyclopropane-1-carboxylique dans 20 $cm^3$ de chlorure de methylène on introduit à 0°C, 1,22 g de dicyclohexylcarbodiimide, quelques cristaux de 4-diméthylaminopyridine, agite pendant 15 minutes à 0°C, introduit une solution de 1,46 g de S cyano (3-phénoxyphényl) méthanol dans 7 $cm^3$ de chlorure de méthylène, agite pendant 5 heures à 20°C, filtre, concentre à sec par distillation sous pression réduite, chromatographie le résidu sur gel de silice en éluant par un mélange de benzène et d'éther de petrole éb. 35°-70°C (6/4) puis par un mélange de benzène et d'éther de pétrole éb. 35°-70°C (7/3) et obtient 754 mg de IR trans 2,2-diméthyl 3=(1,2-propadiényl) cyclopropane-1-carboxylate de S-cyano (3-phénoxy-phényle) méthyle $[\alpha]_D$=-25°(C=0,7%, chloroforme)

**Dichroïsme circulaire** (dioxanne)

Maximum à 237 nm (Δε=-2,8); maximum à 283 nm (Δε= +0,35) inflexion à 287 nm (Δε=+0,32)

**Spectre U.V.** (dans éthanol)

Inflexion vers 228 nm ($E^{1\%}_{1cm}$ : 362) ; inflexion vers 269 nm ($E^{1\%}_{1cm}$ : 50) ; inflexion vers 274 nm ($E^{1\%}_{1cm}$ : 55) ; maximum à 279 nm ($E^{1\%}_{1cm}$ : 60 )

**Microanalyse:**

| | | C% | H% | N% |
|---|---|---|---|---|
| Calculés | : | 76,86 | 5,89 | 3,90 |
| Trouvés | : | 77,0 | 5,9 | 3,8 |

**EXEMPLE 7: 1R trans 2,2-dimethyl 3-(3-ethoxycarbonyl 1,2 butadiényl) cyclopropane carboxylate de (S) cyano (3-phénoxyphényl) methyle**

**Stade A: 1R trans 2,2-diméthyl 3-(2-methoxyéthenyl) cyclopropane carboxylate de terbutyle**

Dans une solution de 59,75 g de (IR trans) 3-formyl 2,2-diméthyl cyclopropane carboxylate de terbutyle dans 1 l de diméthylformamidè et de 106,14 g de chlorure de methoxyméthyltriphénylphosphonium, on introduit à -60°C 33,6 g de terbutylate de potassium en solution dans 450 $cm^3$ de dimethylformamide, agite pendant 30 minutes à -60°C, verse le mélange réactionnel dans l'eau, ajoute de l'éther isopropylique, agite, sépare par decantation la phase organique, extrait à l'ether isopropylique, concentre les phases organiques à sec par distillation sous pression réduite, chromatographie le residu sur silice en éluant avec un mélange d'hexane et d'acétate d'éthyle (9/1) et obtient 49,54 g. IR trans 2,2-diméthyl 3-(E+Z 2-méthoxyéthényl) cyclopropane carboxylate de terbutyle.

**Spectre de RMN**

6,35 - 6,55 ppm }
4,43 à 4,76 ppm } ΔE 3/4

5,98 - 6,1 ppm }
3,98 à 4,25 ppm } ΔZ 1/4

$OCH_3$ → 3,55 ppm
t.Bu → 1,46 ppm

**Stade B: IR trans 2,2-diméthyl 3-formylméthyl cyclopropane carboxylate de terbutyle**

Une solution de 45,26 g de IR trans 2,2-diméthyl 3-(2-méthoxyéthenyl) cyclopropane carboxylate de terbutyle dans 450 $cm^3$ d'acétone contenant 50 $cm^3$ de solution aqueuse 2N d'acide chlorhydrique est agitée pendant 6 heures à 20°C, on concentre à sec, ajoute de l'eau et de l'éther, sépare par décantation la phase organique, extrait à l'éther, concerte les phases organiques à sec par distillation sous pression réduite, chromatographie le résidu sur silice en éluant par un mélange d'hexane et d'acétate d'éthyle (4/1) et obtient 28,75 g de IR trans 2,2-diméthyl 3-formylméthyl cyclopropane carboxylate de terbutyle.

**Spectre de RMN**

1,26 - 1,13 - 1,16 ppm $CH_3$ géminés
1,45 ppm t.Bu
9,9 - 9,95 - 10. ppm H aldéhyde
1,66 à 2,66 ppm $-CH_2-C-$
||
O

**Stade C: 1R trans 2,2-dimethyl 3-carboxyméthyl cyclopropane car-boxylate de terbutyle**

Dans une solution de 25,47 g de IR trans 2,2-diméthyl 3-formylméthyl cyclopropane carboxylate de terbutyle dans 250 $cm^3$ d'eau, on introduit à +10°C, 18 $cm^3$ d'acide sulfurique 66°Bé puis ajoute 14,47 g de permanganate de potassium, agite pendant deux heures à 20°C, ajoute à +10°C du bisulfite de sodium e poudre jusqu'à éécloration du milieu, extrait au dichloromethane, concentre les phases organiques à sec par distillation sous pression réduite.

Le résidu est dissous dans l'acétate d'éthyle on extrait par solution aqueuse de bicarbonate de sodium, réunit les phases aqueuses, les acidifie avec une solution aqueuse concentrée d'acide chlorhydrique, extrait la phase aqueuse acide au dichlorométhane, lave la phaseorganique à l'eau, la sèche, la concentre à sec par distillation sous pression réduite et obtient 15,02 g de 1R trans 2,2-diméthyl 3-carboxymethyl cyclopropane carboxylate de terbutyle.

**Spectre RMN**

1,16 et 1,23 ppm $CH_3$ sur cyclopropyle
1,45 ppm t.Bu
1,15 à 1,76 ppm H du cyclopropyle
2,08 à 2,83 ppm $CH_2CO$
6,75 ppm OH (mobile)

**Stade D: 1R trans 2,2-dimethyl 3-(3-ethoxycarbonyl 1,2 butadié; nyl) cyclopropane carboxylate de terbutyle**

Dans une solution de 7,14 g de 1-éthoxycarbonyl éthylidène triphényl phosphorane dans 30 $cm^3$ de dichlorométhane, on introduit 2,75 $cm^3$ de triethylamine en solution dans 10 $cm^3$ de dichlorométhane, agite pendant 45 minutes à 20°C, (solution A).

par ailleurs dans une solution de 2,9 $cm^3$ de 1-chloro N,N,2-trimethyl propenylamine dans 20 $cm^3$ de dichloromethane on introduit 3,75 g de IR trans 2,2-dimethyl 3-carboxyméthyl cyclopropan-e carboxylate de terbutyle en solution dans 15 $cm^3$ de dichloromethane, agite pendant 15 minutes à 20°C, introduit à -20°C la solution A obtenue précédemment, agite pendant trente minutes à -20°C, verse le mélange réactionnel dans l'eau, extrait au dichlorométhane, concentre les phases organiques à sec par distillation sous pression reduite, chromatographie le residu sur silice en eluant avec un mélange d'hexane et d'acetate d'éthyle-(9/1) et obtient 3,69 g de 1R trans 2,2-diméthyl (3-ethoxycarbo-nyl 1,2.butadiényl) cyclopropane carboxylate de terbutyle.

**Spectre de RMN**

1,13 à 1,41 ppm les $CH_3$ sur cyclopropyle
1,46 ppm t.Bu
5,9 papm = C = C–
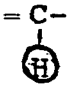
1,86 à 1,91 ppm $H_3C$ - C = C =
4,0 à 4,4 ppm $CH_2$ de l'éthoxy
1,13 à 1,41 ppm $CB_3$ de l'ethoxy

**Stade E: Acide 1R trans 3-(3-éthoxycarbonyl 1,2-butadienyl) 2,2-diméthyl cyclopropane carboxyligue**

On mélange 3,1 g de IR trans -2,2-diméthyl 3-(3-éthoxycarbo-nyl 1,2 hexadiényl) cyclopropane carboxylate de terbutyle, 30 $cm^3$ de benzène, 0,24 g d'acide paratoluène sulfonique, porte le mélange au reflux pendant 30 minutes, verse le mélange réactionnel dans une solution aqueuse contenant 0,23 g de bicarbonate de sodium, extrait au benzène, concentre les phases organiques a sec par distillation sous pression réduite, chromatographie le résidu sur silice en éluant avec un mélange d'hexane et d'acétate d'éthyle (1/1) et obtient 1,54 g d'acide 1R trans 3-(3-éthoxycarbonyl 1,2 butadiényl) 2,2-diméthyl cyclopropane carboxylique.

**Spectre de RMN**

1,13 à 1,41 ppm $CH_3$ sur cyclopropyle
1,5 à 2,16 ppm H du cyclopropyle
1,86 - 1,91 ppm $H_3C$ - C = C= C
4,0 à 4,4 ppm $CH_2$ éthoxy
1,13 à 1,41 ppm $CH_3$ éthoxy

**Stade F: 1R trans 2,2-diméthyl 3,13-éthoxycarbonyl 1,2-butadiényl) cyclopropane carboxylate (S) cyano (3-phénoxyphényl) méthyle**

Dans une solution de 1,5 g d'acide 1R trans 2,2-diméthyl 3-(3-éthoxy carbonyl 1,2 butadiényl) cyclopropane carboxylique dans 30 $cm^3$ de dichlorométhane et de 1,42 g d'alcool (S) cyano (3-phénoxyphényl) méthylique, on introduit 1,3g de dicyclohexylcarbodiimide, 30 mg de 4-diméthylaminopyridine, 15 $cm^3$de dichlorométhane, agite

pendant 18 heures à 20°C, filtre, concentre le filtrat à sec par distillation sous pression réduite, chromatographie le résidu sur silice en éluant avec un mélange d'hexane et d'acétate d'ethyle (4/1) et obtient 1,16 g de IR trans 2,2-dime-thyl 3-(3-éthoxycarbonyl) 1,2-butadiényl cyclopropane carboxylate de (S) cyano (3-phénoxyphényl) méthyle.

$[\alpha]_D = +35°C$ ($CHCl_3$)
Dichroisme circulaire (dioxanne)
Maximum vers 232 nm$\Delta\varepsilon = -2,3$
Maximum vers 283 nm $\Delta\varepsilon = +0,86$
Inflexion vers 288 nm$\Delta\varepsilon = +0,8$

**Spectre U.V.** (éthanol)

Inflexion 266 nm ($E^{1\%}_{1cm}$ : 84) ; maximum 271 nm ($E^{1\%}_{1cm}$ : 85) $\varepsilon$ : 3800
Maximum 277 nm ($E^{1\%}_{1cm}$ : 86) $\varepsilon$ : 3800 ; inflexion 296 nm ($E^{1\%}_{1cm}$: 28)

**Microanalyse:**

| | C% | H% | N% |
|---|---|---|---|
| Calculés | 72,79 | 6,11 | 3,14 |
| Trouvés | 72,6 | 6,0 | 3,0 |

**EXEMPLE 8: Acide IR ci-s 2,2-diméthyl 3-(1,2-propadiényl) cyclopropane carboxylique**

**Stade A: 1R cis 2,2-diméthyl 3-éthynyl cyclopropane carboxylate de méthyle**

Dans une solution de 58,34 g de IR cis 2,2-diméthyl 3(2,2-dibromovinyl) cyclopropane carboxylate de méthyle, on introduit à -100°C, lentement, 100 $cm^3$ de solution 1,85 M de butyllithium dans le cyclohexane, agite pendant 30 minutes à -100°C puis pendant 1 heure à -60°C, verse le mélange réactionnel sur une solution aqueuse glacée de dihydrogéno phosphate de sodium, extrait au benzène, concentre à sec par distillation sous pression réduite, rectifie sous pression réduite et extrait 14,5 g de IR cis, 2,2-diméthyl 3-éthynyl cyclopropane carboxylate de méthyle eb.: 40°C sous 0,5 mm de mercure.

**Spectre de RMN** (deutéro chloroforme)

pics a 1,17 -1,37 ppm attribués aux hydrogènes des methyl géminés pic a 1.72 ppm attribué aux hydrogènes en 1 et 3 du cyclopropyle pics à 2,11 ppm attribués à l'hydrogène de l'éthynyle pic à 3,7 ppm attribué aux hydrogènes du methyle du méthoxycarboxyle.

**Stade B: 1R cis 2,2-dimethyl 3-(1,2-propadienyl) cyclopropane cartoxylate de methyle**

Dans 60 $cm^3$ de dioxane, on introduit 5,78 g de IR cis 2,2-diméthyl 3-(éthynyl)cyclopropane carboxylate de méthyle, 1,79 g de paraformaldéhyde, 6,3 $cm^3$ de diisopropylamine, 1,76 g de bromure cuivreux, porte le mélange réactionnel à 80°C, 1,y maintient pendant 18 heures, retroidit a 20°C, dilue a l'eau, acidifie par une solution aqueuse N d'acide chlorhydrique, extrait au benzène, filtre, concentre à sec sous pression réduite, chromatographie le résidu sur silice en éluant avec un mélange d'ether de pétrole eb. 35°-70°C et de benzène (7/3) et obtient 2,62 g de 1R cis 2,2-diméthyl3-(1,2-propadienyl) cyclopropane carboxylate de methyle.

**Spectre** de RMN (deutéro chloroforme)

pics à 1,17 - 1,25 ppm attribués aux hydrogènes des méthylesgeminés
pics à 1,6 - 1,9 ppm attribués aux hydrogènes du cyclopropyle
pic à 3,6 ppm attribué aux hydrogènes du méthyle du méthoxycarbonyle
pics à 4,7 - 4,8 ppm attribués aux hydrogènes de $CH_2=$
pics à 5,3 - 5,8 ppm attribués à l'hydrogène de =C= 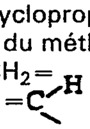

**Stade C: Acide 1R cis 2,2-diméthyl 3-(1,2 propadiényl) cyclopropane carboxylique**

Dans 40 $cm^3$ de méthanol et 20 $cm^3$ de solution aqueuse N de soude, on introduit 2,56 q de IR cis 2,2 diméthyl 3-(1,2 propadienyl) cyclopropane carboxylate de méthyle, porte au reflux du methanol, sous agitation, maintient le reflux pendant 17 heures, refroidit à 20°C, lave au chlorure de méthylène, acidifie la phase aqueuse à $pH_1$, extrait au chlorure de méthylène, concentre à sec par distillation sous pression réduite et obtient 2,15 g d'acide 1R cis 2,2 diméthyl 3-(1,2-propadiényl) cyclopropane carboxylique $[\alpha]_D = +72°$ (c = 0,5%, chloroforme)

**Spectre de RMN**

1,18 et 1,25 ppm $CH_3$ sur cyclopropyle
1,58 à 2,05 ppm H du cyclopropyle
4,7 - 4,8 ppm $H_2C=C=$
5,3 à 5,6 ppm $\textcircled{H}C=C=CH_2$

**EXEMPLE 9: Acide 1R cis 2,2-diméthyl 3-(1,2 propadiényl) cyclopropane carboxyligue**

**Stade A: 1R cis 2,2-diméthyl 3-(1,2-propadiényl) cycloeropane carboxylate de terbutyle**

Dans 1,300 1 de dioxane, on introduit 131 g de 1R cis 2,2 diméthyl 3-(éthynyl)cyclopropane carboxylate de terbutyle, 32 g de paraformaldéhyde, 111 $cm^3$ de diisopropylamine, 31 g de bromure cuivreux, porte la température du mélange réactionnel à 80°C, le maintient pendant 16 heures 30, refroidit à 20°C, ajoute de l'eau acidifie à pH= 1 par addition de solution aqueuse N d'acide chlornydrique, extrait au chlorure de méthylène, lave les phases organiques à l'eau, concentre à sec par distillation sous pression reduite, chromatographie le résidu sur silice en éluant avec un mélange de chlorure de méthylène et d'hexane (1/1) et obtient 44 g de 1R cis 2,2-diméthyl 3-(1,2-propadiényl) cyclopropane carboxylate de terbutyle.

**Spectre de RMN** (deutéro chloroforme)

pics à 1,2 - 1,27 ppm attribués aux hydrogènes desméthyle géminés
pic à 1,48 ppm attribué aux hydrogènes de terbutyle
pics à 4,7 - 4,8 ppm attribués aux hydrogènes de $=CH_2$ pics à 5,36 - 5,75 ppm attribués à l'hydrogène de -CH=C=

**Stade B: Acide 1R cis 2,2-diméthyl 3-(1,2-propadiényl) cyclopropane carboxylique (seconde préparation)**

Dans 990 $cm^3$ de solution éthanolique N de potasse, on introduit 82,4 g de IR cis 2,2-diméthyl 3-(1,2-propadiényl) cyclopropane carboxylate de terbutyle, porte le mélange réactionnel au reflux, l'y maintient pendant 16 heures, le refroidit à -20°C, verse le mélange réactionnel dans l'eau, extrait au chlorure de méthylène, concentre à sec par distillation sous pression reduite, acidifie la phase aqueuse, extrait au chlorure de méthylène, lave à l'eau, concentre à sec par distillation sous pression réduite, chromatographie le résidu sur gel de silice en éluant dans l'éther isopropylique et recueille 39,5 g d'acide IR cis 2,2-diméthyl 3-(1,2-propadiényl) cyclopropane carboxylique $[\alpha]_D = +86°$ (c=6,5%, chloroforme)

**Spectre de RMN** (deutero chloroforme)

pics à 1,23 - 1,3 ppm attribués aux hydrogènes desméthylesgeminés
pics de 1,6 à 2,05 ppm attribués aux hydrogènes du cyclopropyle
pics à 4,7 - 4,9 ppm attribués aux hydrogènes de $CH_2=$
pics à 5,3 à 6,5 ppm attribués à l'hydrogène de -CH=C=
pics à 10,6 ppm attribués à l'hydrogène du carboxyle.

**EXEMPLE 10: Acide IR trans 2,2-dimethyl 3-(1,2 propadienyl)**

cycloprane carboxylique>1

**Stade A: 1R trans 2,2-diméthyl 3-éthynyl cycloeropane carboxylate de terbutyle**

Dans 200 $cm^3$ d'éther éthylique et 200 $cm^3$ de tétrahydrofurane, on introduit 38,7 g de IR trans 2,2-diméthyl-3-(2,2-dibromovinyl) cyclopropane carboxylate de terbutyle, introduit lentement à -100° C, 47 $cm^3$ de solution 2,34 M de butyllithium dans l'hexane, agite pendant 1 heure et trente minutes à -80° C, verse le mélange réactionnel sur une solution aqueuse glacée de dihydrogéno phosphate de ocdium, extrait au tenzène, concentre à sec par distillation sous pression réduite, rectifie sous pression réduite et obtient 9,3 g de IR trans 2,2-diméthyl 3-éthynyl cyclopropane cartoxylate de terbutyle, eb. 30° C sous 0,1 mm de mercure.

**Spectre de RMN** (deutéro chloroforme)

pics à 1,22 - 1,3 ppm attribués aux hydrogènes des méthyles gemines
pic à 1,47 ppm attribué aux hydrogènes du terbutyle
pics à 1,57 - 1,65 ppm attribués à l'hydrogène en position 1 du cyclopropyle
pics de 1,78 à 1,96 ppm attribués à l'hydrogène en 3 du cyclopropyle et à l'hydrogène de l'éthynyle.

**Stade B: (1R trans) 2,2-diméthyl 3-(1,2-propadiényl) cyclopropane carboxylate de terbutyle**

Dans 100 $cm^3$ de dioxane, on introduit 9,2 g de IR trans 2,2-diméthyl 3-éthynyl cyclopropane carboxylate de terbutyle, 2,3 g de paraformaldéhyde, 8 $cm^3$ de diisopropylamine, 2,3 g de bromure cuivreux, porte le mélange réactionnel à 90° C, l'y maintient pendant 51 heures, refroidit à 25° C, dilue à l'eau, filtre, extrait au benzène, concentre à sec par distillation sous pression réduite, chromatographie le résidu sur gel de silice en eluant avec un mélange d'éther de pétrole éb. 35°-70° et de benzène (7/3) et obtient 3,95 g de IR trans 2,2-diméthyl 3-(1,2-propadiényl) cyclopropane carboxylate de terbutyle

**Spectre de RMN** (deutéro chloroforme)

pics à 1,13 - 1,23 ppm attribués aux hydrogènes des méthylesgéminés
pic a 1,45 ppm attribué aux hydrogènes du terbutyle
pics à 4,6 à 5,3 ppm attribués aux hydrogènes de $-CH=C=CH_2-$

**Stade C: Acide IR trans 2,2-dimethyl 3-(1,2-propadiényl) cyclopropane carboxylique**

Dans 40 $cm^3$ de toluène et 400 mg d'acide paratoluène sulfo: nique, on introduit 3,9 g de IR trans 2,2-diméthyl 3= (1,2-propa-diényl) cyclopropane carboxylate de terbutyle, porte le mélange réactionnel à 120° C, l'y maintient pendant 30 minutes, refroidit à 20° C, lave à l'eau, extrait au benzène, concentre à sec par distillation sous pression réduite et obtient 2,71 g d'acide IR trans 2,2-diméthyl 3-(1,2-propadiényl) cyclopropane carboxylique
$[\alpha]_D = -12,5°$ (C=0,5%, chloroforme)

**Spectre de RMN** (deutéro chloroforme)

pics à 1,15 - 1,23 ppm attribués aux hydrogènes des méthyles géminés
pics a 1,46 - 1,55 ppm attribués à l'hydrogène en position 1 du cyclopropyle
pic à 1,97 ppm attribué à l'hydrogène en position 3 du cyclopropyle,
pics de 4,7 à 5,3 ppm attribués aux hydrogènes du propadiényle pic a 10,8 ppm attribué à l'hydrogène du carboxyle.

**EXEMPLE 11: 1R cis 2,2-diméthyl 3-(3-éthoxy carbonyl) 1,2 propadienyl) cyclopropane carboxylate de (S) cyano (3 phénoxyphényl) méthyle**

**Stade A: Acide 1R cis 2,2-diméthyl 3-(2-méthoxy 1-éthenyl) cyclopropane carboxylique**

Dans une solution renfermant 12,58 g de sel de sodium de l'acide 1R, cis 2,2-dimethyl 3-formyl cyclopropane carboxylique, 350 $cm^3$ de diméthylformamide et 26,27 g de chlorure de méthoxy méthyl triphényl phosphonium, on introduit à -60°C 9 g de terbutylatede potassium à 96,5% en solution dans 90 $cm^3$ de diméthylformamide, on agite en laissant remonter la température jusqu'à 20°C en une heure trente minutes, verse le mélange réactionnel dans une solution aqueuse de phosphate monosodique, extrait à l'oxyde de diisopropyle, réunit les phases organiques, les extrait avec 100 $cm^3$ de solution aqueuse N de soude, agite la phase aqueuse à +5°C avec un mélange d'oxyde de diisopropyle et de phosphate monosodique, dilue à l'eau, sépare par décantation la phase organique, extrait la phase aqueuse à l'oxyde de diisopropyle, concentre les phases organiques à sec par distillation sous pression reduite et obtient 5,22 g d'acide IR cis (E +Z) 2,2-diméthyl3-(2-methoxy 1-éthényl) cyclopropane carboxylique

**Spectre de RMN**

6,4 - 6,6 ppm } ΔE 3/4
4,9 - 5,3 ppm }
4,6 - 4,8 PPM }
4,7 - 4,9 ppm } ΔZ 1/4
et
6,0 à 6,2 ppm }
} Protons éthyléniques

3,5 - 3,6 - 3,66 ppm $CH_30$
1,13 - 1,21 - 1,28 ppm les $CH_3$ sur cyclopropyl

**Stade B: 1R cis 2,2-diméthyl 3-12-méthoxy 1 (SE + Z) éthényl) cycloeropane carboxylate de (S) cyano (3-phénoxyphényl) methyle**

Dans une solution renfermant 5,22 q d'acide IR cis 2,2-diméthyl 3-(2-méthoxy 1-éthényl) cyclopropane carboxylique, 50 $cm^3$ de dichlorométhane et 6,9 g d'alcool (S) cyano (3-phenoxyphenyl) méthylique, on introduit à 0°C une solution de 6,32 g de dicyclohexylcarbodiimide, et 0,22 g de 4-diméthylaminopyridine dans 50 $cm^3$ de dichlorométhane. On agite pendant 2 heures à température ambiante, filtre, concentre le filtrat à sec par distillation sous pression reduite, chromatographie le résidu sur silice avec un mélange d'hexane et d'acetate d'ethyle (9/1) et obtient 8,18 g de 1R cis 2,2-diméthyl 3-(2-méthoxy 1 (E + Z) éthényl) cyclopropane carboxylate de (S) cyano (3-phenoxyphényl) méthyle

**Spectre de RMN**

4,8 - 5,0 ppm } isomère E
5,0 - 5,2 ppm }
4,5 - 4,7 ppm } isomère Z
4,7 - 4,8 ppm }
} Proton éthylénique en β de $OCH_3$
6,4 - 6,7 ppm isomère E
6,0 - 6,2 ppm isomère Z
} . = 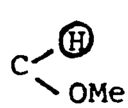

3,56 ppm isomère ΔE } $H_3CO$
3,66 ppm isomère ΔZ }
6,4 ppm 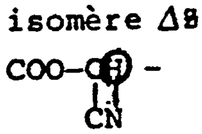

51,18 a 1,25 ppm les $CH_3$ gémines.

**Stade C: 1R cis 2,2 -diméthyl 3-formylméthyl cyclopropane carboxylate de (S) cyano (3-phénoxyphényl) méthyle**

Une solution de 1,9 g de IR cis 2,2-diméthyl 3-(2-méthoxy 1 (E + Z) éthényl) cyclopropane carboxylate de (S) cyano (3-phénoxyphényl) méthyle, 20 cm3 d'acétone et 2,5 cm3 de solution aqueuse 2N d'acide chlorhydrique est agitée à 20°C pendant 18 heures. On concentre à sec par distillation sous pression réduite, ajoute de l'eau,

extrait à l'acétate d'éthyle, concentre les phases organiques à sec par distillation sous pression réduite, chromatographie le résidu sur silice en eluant avec un mélange d'hexane et d'acétate d'ethyle (4/1) et obtient 1,3 g de IR cis 2,2-diméthyl 3-formylméthyl cyclopropane carboxylate de (S) cyano 3-(phénoxyphényl) méthyle.

**Spectre de RMN**

1,11 et 1,23 ppm $CH_3$ géminés

2,85 - 2,96 ppm 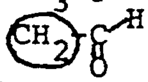

9,8 ppm H du formyle

1,5 à 1,75 ppm H du cyclopropyle

6,2 ppm (H) - C -
CN

6,9 à 7,6 ppm les protons aromatiques

**Stade D: 1R cis 2,2-diméthyl 3-carboxymethyl cyclopropane carboxylate de (S) cyano (3-phénoxyphenyl) méthyle**

Dans un mélange de 1,25 g de 1R cis 2,2-diméthyl 3-formyl méthyl cyclopropane carboxylate de (S) cyano (3-phénoxyphényl) méthyle et de 10 $cm^3$ d'eau, on introduit à 0°C, goutte à goutte, 0,5 $cm^3$ d'acide sulfurique 66°Bé, agite pendant 5 minutes, ajoute 0,46 g de permanganate de potassium, agite pendant 2 heures en laissant monter la température à 20°C, additionne de bisulfate de sodiumen poudre jusqu'à décoloration, ajoute de l'éther éthylique et du chlorure de sodium, extrait à l'éther, concentre les phases ethérées à sec après sechage sur $SO_4Na_2$ par distillation sous pression réduite, chromatographie le résidu sur silice en éluant avec un mélange d'hexane et d'acétate d'éthyle (1/1) et obtient 0,98 g de 1R cis 2,2-diméthyl 3-carboxyméthyl cyclopropane carboxylate de (α) cyano (3-phénoxyphényl) méthyle

**Spectre de RMN**

1,16 et 1,28 ppm les $CH_3$ géminés

2,7 à 2,8 ppm $CH_2$-COO

1,5 à 1,83 ppm les H du cyclopropyle

6,3 ppm - COO - C(H) -
CN

6,9 à 7,6 ppm les H aromatiques.

**Stade E: 1R cis 2,2-diméthyl 3-(3-ethoxy carbonyl) 1,2 propadiényl cyclopropane carbboxylate de (S) cyano (3-phénoxy phényl) méthyle**

Dans une solution de 1,54 g de carbéthoxy méthylène triphényl phosphorane dans 10 $cm^3$ de dichlorométhane anhydre, on introduit 0,62 $cm^3$ de triéthylamine et 5 $cm^3$ de dichlorométhane, agite pendant 30 minutes à 20°C, et obtient la solution A.

Par ailleurs dans une solution de 0,65 $cm^3$ de 1-chloro N,N, 2-trimethyl propénylamine dans 10 $cm^3$ de dichlorométhane on introduit 1,4 g de IR cis 2,2-diméthyl 3-carboxyméthyl cyclopropane carboxylate de (S) cyano (3-phenoxyphenyl) méthyle en solution dans 10 $cm^3$ de dichlorométhane, introduit à -50°C la solution A obtenue précédemment, agite pendant une heuréet 30 minutes à -50°C, verse le mélange réactionnel dans l'eau, extrait deux fois au dichlorométhane, concentre les phases organiques à sec par distillation sous pression réduite, chromatographie le résidu sur silice en éluant par un mélange d'hexane et d'acétate d'éthyle (7/3) et obtient 0,78 g de 1R cis 2,2-diméthyl 3-(3-éthoxycar-bonyl) 1,2-propadiényl cyclopropane carboxylate de (S) cyano (3-phénoxyphényl) méthyle.

**Spectre de RMN**

1,23 - 1,26 - 1,3 ppm $CH_3$ géminés et $CH_3$ de l'éthoxy

5,63 à 6,13 ppm H alléniques

4,05 - 4,16 - 4, 28 - 4,4 ppm $CH_2$ de l'éthoxy

- 6,4 ppm (H) - C -
CN

7,0 à 7,6 ppm les protons aromatiques

**Dichroisme circulaire** (dioxanne)

maximum 217 nm Δ ε= + 13,8
maximum 267 nm Δ ε= + 0,38
maximum 282 nm Δ ε= + 0,39
inflexion 285 Δε= + 0,36

**Microanalyse:** C% H% N%

Calculés 72,37 5,84 3,25
Trouvés 72,1 5,9 3,3
$[\alpha]_D$ = +40° c = 0,5% dans $CHCl_3$

**EXEMPLE 12: 1R trans 3-(1,2-propadiényl) 2,2-dimethyl cyclopropane carboxylate d' (RS) cyano 6'phénoxy 2'pyridylmethyle**

On mélange 2 g d'acide 1R trans 2,2-diméthyl 3-(1,2-propadienyl) cyclopropane carboxylique, 20 cm³ de chlorure de méthylène, 2,9 g d'alcool (RS) cyano 6'phénoxy 2'pyridylméthylique, 100 mg de 4-diméthyl aminopyridine, on introduit à 0°C 2,88 g de dicyclohexylcarbodiimide en solution dans 30 cm³ de chlorure de méthylène, agite pendant 16 heures à 20°C, filtre, concentre le filtrat à sec par distillation sous pression reduite, chromatographie le residu sur silice en éluant par un mélange d'hexane et d'acétate d'éthyle (8/2) et obtient 3,84 g de IR trans 3-(1,2 propadienyl)-2,2-dimethyl cyclopropane carboxylate (RS) cyano 6'phénoxy 2' pyridyl méthyle$[\alpha]_D$ = -7° (c = 1% chloroforme)

**Dichroisme circulaire** (dioxanne)

maximum 215 nm Δε = + 3,6
maximum 235 - 236 nm Δε = - 3,5
maximum 265 nm Δ ε = - 0,26
maximum 292 nm Δε= - 0,07

**Microanalyse** C% H% N%

Calculés 73,31 5,59 7,77
Trouvés 13,2 5,5 7,6

**EXEMPLE 13: 1R trans 3-(1,2-propadiényl) 2,2-diméthyl cyclopropane carboxylate de pentaflouorophénylméthyle**

Dans un mélange de 3 g d'acide 1R trans 2,2-diméthyl 3-(1,2-propa-diényl) cyclopropane-1-carboxylique, de 30 cm³ de chlorure de methylène, de 3,96 g d'alcool pentafluorobenzylique et de 100 mg de 4-diméthyl aminopyridine, on introduit à 0°C 4,12 g de dicyclohexylcarbodiimide en solution dans 40 cm³ de chlorure de méthylène, agite pendant 16 heures à 20°C, filtre, concentre le filtrat à sec par distillation sous pression reduite, chromatographie le résidu sur silice en éluant avec un mélange d'hexane et d'acétate d'éthyle (9/1) et obtient 4,78 g de 1R trans 3-(1,2-propadiényl) 2,2-diméthyl cyclopropane carboxylate de pentafluorophényl méthyle. $[\alpha]_D$= - 23° (C=0,5%, chloroforme)

**Dichroïsme circulaire** (dans dioxanne)

maximum 234 nm Δε = - 20,1
maximum 266 nm Δε= - 0,05

**Microanalyse** C% H% F%

Calcules 57,83 3,94 28,59
Trouvés 58,1 4,1 28,2

**EXEMPLE 14: 1R trans 3-(1,2-propadiényl) 2,2diméthyl cyclopropane carboxylate de (1-(3-propyn-2-yl) 2,5-dioxo imidazolidinyl) méthyle**

Dans une solution de 2 g d'acide 1R, trans 3-(1,2-propa-diényl) 2,2-diméthyl cyclopropane-carboxylique, de 20 $cm^3$ de chlorure de méthylène de 100 mg de 4-diméthylaminopyridine et de 2,2 g de 2,5-dioxo 1-hydroxyméthyl 3-(2-propynyl) imidazole, on introduit à 0°C 2,88 g de dicyclohexylcarbodiimide en solution dans 30 $cm^3$ de chlorure de méthylène, agite pendant 16 heures à 20°C, filtre, concentre le filtrat à sec par distillation sous pression réduite, chromatographie le résidu sur silice en éluant avec un mélange d'hexane et d'acetate d'éthyle (1/1) et obtient 1,6 g de IR trans, 3-(1,2-propadiényl) 2,2-diméthyl cyclopropane carboxylate de (1(3-propyn-2-yl) 2,5-dioxo imidazolidinyl) méthyle.

**Analyse** $C_{16}H_{18}N_2O_4$ (302,32)

C% H% N%
Calculés 63,56 6,00 9,27
Trouvés 63,3 6,0 9,1
$[\alpha]_D = -27°$ c= 1% $CHCl_3$

**Dichroisme circulaire** (dans dioxanne)

maximum 215 nm $\Delta\varepsilon = +2,0$
maximum 235 nm $\Delta\varepsilon = -2,8$
maximum 275 nm $\Delta\varepsilon = +0,007$
maximum 292 nm $\Delta\varepsilon = -0,007$

**Exemple 15: 1R cis 2,2-diméthyl 3- (1, 2-propadien-yl-)—cyclopropane carboxylate de (S) cyano 1-5-3-phénoxyphényl) méthyle.**

En opérant comme à l'exemple 1 en utilisant l'alcool (S) cyano (3-phénoxyphényl) méthylique, on obtient le produit attendu.
$/\alpha/D = +54°$ (c = 0,5% CHCl3).

**Exemple 16: 1R cis 2,2-diméthyl 3- (1,2-propa-diényl) cyclopropane carboxylate de /-1-5-3-propyn-2-yl) 2,5-dioxo imidazolidinyl/ methyle.**

En opérant comme à l'exemple 1 en utilisant l'alcool /1-(3-propyn-2-yl) 2,5-dioxo imidazolidinyl/ méthylique, on obtient le produit attendu.
$/\alpha/_D = +14,5° + 2°$ (c = 0,4% $CHCl_3$).

**Exemple 17: 1R cis 2,2-diméthyl 3-(1,2-propadiényl) cyclopropane carboxylate de pentafluorophenylméthyle.**

En opérant comme à l'exemple 1 en utilisant l'alcool pentafluorophenyl méthylique, on obtient le produit attendu. $/\alpha/_D = +20,5° \pm 1°$ (c = 1,5% $CHCl_3$).

**Exemple 18: 1R cis 2,2-diméthyl 3- (1,2-propadienyl) cyclopropane carboxylate de (RS) cyano 6-phénoxy 2-pyridyl méthyle.**

En operant comme a l'exemple 1 en utilisant l'alcool α(RS) cyano 6-phénoxy 2-pyridyl méthylique, on obtient le produit attendu.
$/\alpha/_D = +59° \pm 2,5°$ (c = 0,6% $CHCl_3$).

**Exemple 19: 1R cis 2,2-diméthyl 3- (1,2-propadiényl) cyclopropane carboxylate de (S) cyano 1-(3-phénoxy 4-fluorophényl) méthyle.**

On opérant comme à l'exemple 1 en utilisant l'alcool (S) cyano 1-(3-phénoxy 4-fluorophényl) méthylique, on obtient le produit attendu.
$/\alpha/_D = +58° \pm 1,5°$ (c = 1% $CHCl_3$).

**Exemple 20: 1R trans 2,2-dimethyl 3-(1,2-propadiényl) cyclopropane carboxylate de (S) cyano 1-(3-phénoxy 4-fluorophenyl) methyle.**

On opérant comme à l'exemple 3 en utilisant l'alcool (S) cyano 1-(3-phénoxy 4-fluorophényl) méthylique, on obtient le produit attendu.
$/\alpha/_D = -19° \pm 2°$ (c = 0,5% $CHCl_3$).

**Exemple 21: Acide 1R trans 2,2-diméthyl 3-(54-ethoxy 4-oxo 1,2-butadiényl) cYclopropane carboxylique.**

**Stade A: IR trans 3-(4-éthoxy 4-oxo 1,2-butadiényl) 2,2-diméthyl cyclopropane carboxylate de terbutyle.**

On opère comme à l'exemple 7, Stade D, en utilisant comme réactif le 1-éthoxy carbonyl méthylène triphényl phosphorane et en remplaçant le dichorométhane par le chlorure de méthylène. On obtient le produit attendu.

**Spectre de RMN:** ($CDCl_3$).

1,15 à 1,53 ppm: les $CH_3$ du cyclopropyle
1,46 ppm: t.Bu.
5,41 à 5,83 ppm: les hydrogènes de la chaîne allénique
4,0 à 4,35 ppm: $CH_2$ de l'éthoxy

**Stade B: Acide 1R trans 2,2-dimethyl 3-(4-éthoxy 4-oxo 1,2-butadiényl) cyclopropane carboxylique.**

On porte au reflux 2,5 g d'ester obtenu au stade A dans 50 cm3 de toluène et ajoute 0,25 g d'acide paratoluène sulfonique. Après 10 minutes de reflux, on amène à sec sous pression réduite, chromatographie sur silice en éluant par un mélange (hexane-acétate d'éthyle 4/6) et obtient 1,4 g de produit attendu.

**Spectre de RMN**

1,16 à 1,33 ppm: $CH_3$ du cyclopropyle
4,05 à 4,4 ppm: $CH_2$ de l'éthoxy
5,5 à 5,83 ppm: H de la chaîne allénique
10,4 ppm: $CO_2H$

**Exemple 22: 1R trans 2,2-diméthyl 3-(4-éthoxy 4-oxo 1,2-butadiényl cyclopropane carboxylate de (S) cyano 1 (3-phénoxylpheél) méthyle - isomè re A et isomè re B.**

On porte 2 heures au reflux 2,2 g d'acide préparé comme au stade B de l'exemple 21 dans 25 cm3 de chlorure de méthylène et 5 cm3 de chlorure de thionyle. On concentre à sec sous pression réduite, reprend l'huile au benzène et concentre à sec de nouveau. On reprend le chlorure d'acide dans 20 cm3 de benzène et ajoute 2,25 g d'alcool (S) cyano (3-phénoxyphényl) méthylique, refroidit à +10°C et ajoute lentement 1 cm3 de pyridine. Après 2 heures d'agitation, on verse le mélange réactionnel dans l'acide chlorhydrique N, décante, lave la phase organique à l'eau, seche, concentre à sec et chromatographie sur silice en éluant par le mélange hexane-acétate d'éthyle (8/2). On obtient 0,72 g d'isomère A et 0,38 g d'isomère B de produit attendu.

**Spectre de RMN**

Isomère A
1,25 ppm: $CH_3$ du cyclopropane
5,5 à 5,83 ppm: H de 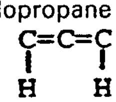
6,4 ppm: H de CH-CN
1,15 à 1,38 et 4,05 à 4,4 ppm = $C_2H_5$
Isomère B
1,22 à 1,25 ppm: $CH_3$ du cyclopropane
5,33 à 5,83 ppm: H de 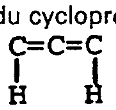
6,4 ppm: H de CH-CN
1,18 à 1,42 et 4,05 à 4,4 ppm: $C_2H_5$

**Exemple 23: 1R trans, l'S 2,2-dimethyl 3-(4-éthoxy 4-oxo 1,2butadiényl) cyclopropane carboxylate de 3-allyl 2-méthyl 4-oxo 2-cyclopenten-1-yle.**

On opère comme à l'exemple 22 en utilisant 1,5 g de (S) 3-allyl 2-méthyl 1-hydroxy 2-cyclopenten-4-one. On obtient 0,794 g de produit attendu.

**Spectre de RMN**

1,23 à 1,3 ppm: $CH_3$ du cyclopropane
5,5 à 6,25 ppm: H de la chaîne allénique et en 1 de l'alléthrolone
1,16 à 1,4 et 4,03 à 4,38 ppm: $C_2H_5$
4,83 à 5,16 ppm: $CH_2$ de la chaîne allylique
5,5 à 6,25 ppm: CH de la chaîne allylique

**Exemple 24: 1R cis 2,2-dimethyl 3-/(3-éthoxycarbonyl) 1,2-propadiényl/ cyclopropane carboxylate de terbutyle.**

**Stade A: IR cis 2,2-diméthyl 3-carboxyméthyl cyclopropane carboxylate de terbutyle.**

On opère comme aux stades A, B et C de l'exemple 7 en utilisant au départ le IR cis 3-formyl 2,2-diméthyl cyclopropane carboxylate de terbutyle. On obtient le produit attendu.

**Stade B: IR cis 2,2-diméthyl 3-/(3-éthoxycarbonyl) 1,2-propadiényl/ cyclopropane carboxylate de terbutyle.**

On opère comme à l'exemple 7, stade D en utilisant le produit obtenu au stade A de l'exemple 24 et comme réactif le bromure de carbéthoxy méthyl triphényl phosphonium. On obtient le produit attendu.

**Spectre de RMN** ($CDCl_3$)

1,22 à 1,48 ppm: 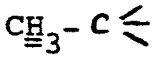
1,52 ppm: t.Bu.
4,2 à 4,57 ppm: $CH_2$ de 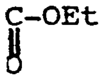
5,45 à 6,23 ppm: H éthyleniques
5,6-5,7 ppm: 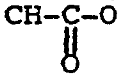

**Exemple 25-: 1R trans 2,2-diméthyl 3-/(3-terbutoxycarbonyl) 1,2propadiényl/ cycloprpane carboxylate de (S) cyano (3-phénoxyphenyl) méthyle.**

**Stade A: 1R trans 2,2-diméthyl 3-carboxyméthyl cyclopropane**

**carboxylate de (S) cyano (3-phenoxyphenyl) méthyle.**

On opère comme aux stades A B C et D de l'exemple 11 en utilisant au depart le sel de sodium de l'acide IR trans 2,2-diméthyl 3-formyl cyclopropane carboxylique et obtient le produit attendu.

**Stade B: IR trans 2,2-diméthyl 3-/(3-terbutoxycarbonyl) 1,2propadiényl/ cyclopropane carboxylate de (S) cyano (3-phénoxyphényl) méthyle.**

On opère comme à l'exemple 11 stade E, en utilisant le produit obtenu au stade A de l'exemple 25 et comme réactif le 1-terbutoxycarbonyl méthylène triphényl phosphorane. On obtient le produit attendu.

**Spectre de RMN**

1,05 à 1,12 ppm: $CH_3$
2,9-2,92 ppm: $CH_2$ en α du cyclopropyle
6,43 ppm: -CH-CN

**Exemple 26: 1R trans 2,2-diméthyl 3-(3-bromo 3-méthoxycarbonyl 1,2-propadiényl) cyclopropane carboxylate de (S) cyano (3-phénoxyphényl) méthyle.**

On opère comme à l'exemple 11 stade E, en utilisant le produit obtenu au stade A de l'exemple 25 et comme réactif le 1-méthoxycarbonyl 1-bromo triphényl phosphorane. On obtient le produit attendu.

**Spectre de RMN** ($CDCl_3$)

1,25 ppm: $CH_3$ gémines
3,83 à 3,85 ppm: $CO_2CH_3$
5m63 à 5,83 ppm: H de la chaîne allénique
6,41 ppm: -CH-CN
6,97 à 7,58 ppm: aromatiques.

**Exemple 27: Acide 1R trans 2,2-diméthyl 3-(3-chloro 1,2-propadiényl) cyclopropane carboxylique.**

**Stade A: IR trans 2,2-diméthyl 3-(RS hydroxy 2-propynyl) cyclopropane carboxylate de terbutyle.**

On refroidit à +10°C une solution de bromure d'éthynyl magnésium et coule lentement 15,04 g de IR trans 2,2-diméthyl 3-formyl cyclopropane carboxylate de terbutyle dans 30 cm3 de tétrahydrofuranne. On agite pendant 2 heures puis verse le melange réactionnel sur une solution aqueuse de phosphate monosodique. On extrait à l'oxyde de diéthyle, concentre à sec les phases organiques sous pression réduite et récupère l'huile que l'on chromatographie sur silice (éluant: benzène-acétate d'éthyle (9/1)). On obtient 17,17 g de produit attendu.

**Stade B: IR trans 2,2-diméthyl 3-(3-chloro 1,2-propadiényl) cyclopropane carboxylate de terbutyle.**

On refroidit à -60°C, 11,26 g de produit obtenu au stade précédent dans 110 cm3 de tétrahydrofuranne puis coule en 20 minutes 5,58 g de terbutylate de potassium dans 75 cm3 de tétrahydrofuranne. On agite pendant 25 minutes puis ajoute lentement 9,4 g de chlorure de tosyle dans 30 cm3 de tétrahydrofuranne. Après 40 minutes d'agitation, on coule en 45 minutes 500 cm3 d'une solution décimolaire de chlorure mixte de cuivre et de lithium dans le tétrahydrofuranne. On agite encore 90 minutes à -60°C puis verse le mélange réactionnel dans une solution aqueuse saturée de chlorure d'ammonium. On extrait à l'oxyde de diéthyle, concentre à sec les phases organiques réunies et recueille 19,5 g de produit que l'on purifie par chromatographie sur silice en eluant par un mélange hexane-oxyde de diisopropyle (95/5). On obtient 1,47 g de produit attendu.

**Spectre de RMN** ($CDCl_3$)

1,17 et 1,27 ppm: $CH_3$ du cyclopropyle
1,46 ppm: t.Bu.
5,38 à 5,7 ppm: C=C=CH
6,0 à 6,2 ppm: C=C=CH-Cl

**Stade C: Acide IR trans 2,2-diméthyl 3-(3-chloro 1,2-propadienyl) cyclopropane carboxylique.**

On chauffe. 30 minutes à 120°C 1,44 g du produit obtenu au stade précédent dans 20 cm3 de toluène et 0,14 g d'acide paratoluène sulfonique. On laisse revenir à température ambiante, verse dans une solution de bicarbonate de sodium, extrait à l'acétate d'éthyle, concentre à sec les phases organiques réunies, recupère 1,19 g de produit brut que l'on purifie par chromatographie sur silice (éluant: hexane-acetate d'éthyle (8/2)). On obtient 0,64 g de produit attendu.

**Spectre IR** ($CHCl_3$)

DH acide mono + dimère 3510 $cm^{-1}$
C=0 acide mono 1730 $cm^{-1}$ epaulement 1695 $cm^{-1}$ maxi
$CB_3$ gémines 1380 $cm^{-1}$
allène 1956 cm

**Exemple 28: 1R trans 2,2-diméthyl 3-(3-chloro 1,2-propadiényl) cyclopropane carboxylate de (S) cyano (3-phénoxyphényl) methyle.**

On opère comme à l'exemple 1 en utilisant 0,6 g d'acide obtenu au stade C de l'exemple 27 et 0,73 g d'alcool (S) cyano (3-phénoxyphényl)méthylique. On obtient 1,0 g de produit attendu.
$/\alpha/_D = +26° \pm 2°$ (c = 0,75% $CHCl_3$)

**Exemple 29: Acide 1R trans 2,2-diméthyl 3-(3-bromo 1,2-propadiényl) cyclopropane carboxyligue.**

**Stade A: IR trans 2,2-diméthyl 3-(3-bromo propadiène) cyclopropane carboxylate de terbutyle.**

On opere comme au stade B de l'exemple 27 en utilisant 13 55 g de produit préparé au stade A de l'exemple 27 et comme réactif 160 cm3 d'une solution demi molaire de bromure de cuivre et de lithium dans le tétrahydrofuranne. On obtient 5,25 g de produit attendu.

**Spectre de RMN** ($CDCl_3$)

1,2 et 1,27 ppm: $CH_3$ du cyclopropyle
1,47 ppm: t.Bu.
5,17 à 6,67 ppm: éthyléniques complexes

**Stade B: Acide 1R trans 2,2-diméthyl 3-(3-bromo 1,2-propadiényl) cyclopropane carboxylique.**

On opère comme au stade C de l'exemple 27 en utilisant 6 g de 1 ester preparé comme au stade A de l'exemple 29. Après chromatographie sur silice (éluant: hexane-acétate d'éthyle (7/3) à 1‰ d'acide acétique), on obtient 4,1 g de produit attendu.

**Spectre de RMN**

1,24 et 1,35 ppm: $CH_3$ géminés
1,59-1,67 et 1,61-1,71 ppm: B du cyclopropyle en α de 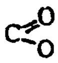 5,2 à 5,75 ppm et 6,02 à 6,25 ppm: H de la chaîne allénique.

**Exemple 30: IR trans 2,2-diméthyl 3-(3-bromo 1,2-propadiényl) cyclopropane carboxylate de (3-phénoxyphényl) méthyle.**

On opère comme à l'exemple 1 en utilisant 1,2 g d'acide obtenu à l'exemple 29 et 1 g d'alcool (3-phénoxyphényl) méthylique. Après chromatographie sur silice (éluant: hexane-acétate d'éthyle (9/1)), on obtient 1,49 g de produit attendu. /α/D = +15,5° ± 2° (c = 0,5% CHCl3)

**Exemple 31: IR trans 2,2-diméthyl 3-(3-chloro 1,2-propadienyl) cyclopropane carboxylate de (3-phenoxyphényl) méthyle.**

On opère comme à l'exemple 30 en utilisant l'acide préparé à l'exemple 27. On obtient le produit attendu.

**Exemple 32: IR trans 2,2-diméthyl 3-(3-bromo 1,2-propadienyl)**

**cyclopropane carboxylate de (S) 2-méthyl 3-allyl 4-oxo 2-cyclopenten-1-yle.**

On opère comme à l'exemple 1 en utilisant 1,38 g d'acide obtenu à l'exemple 29 et 0,9 g de (S) 3-allyl 2-méthyl 1-hydroxy 2-cyclopenten-4-one. Aprés chromatographie sur silice (éluant: hexane-acétate d'éthyle (7/3)), on obtient 1,53 g de produit attendu.
$/\alpha/_D = +7{,}5° \pm 2°$ (c = 0,5% $CHCl_3$)

**Exemple 33: 1R trans 2,2-diméthyl 3-(3-chloro 1,2-propadiényl) cyclopropane carboxylate de (S) 2-méthyl 3-allyl 4-oxo 2-cyclopenten-1-yle.**

On opère comme à l'exemple 32 en utilisant l'acide préparé à l'exemple 27. On obtient le produit attendu.

**Exemple 34: IR trans 2,2-diméthyl 3-(3-bromo 1,2-propadiényl) cyclopropane carboxylate de (S) cyano (3-phénoxyphényl) méthyle.**

On opère comme à l'exemple 1 en utilisant 1,2 g d'acide obtenu à l'exemple 29 et 1,17 g d'alcool (S) cyano (3-phénoxyphényl) méthylique:Après chromatrographie sur silice (éluant: hexane-acétate d'ethyle (9/1) puis hexane-éther isopropylique (8/2)), on obtient 0,55 g de produit attendu. F = 98°C.

**Spectre de RMN**

1,18 et 1,25 ppm: $CH_3$ du cyclopropyle
5,18 à 5,8 ppm et 6,05 à 6,25 ppm:H de la chaîne allénique
6,43 ppm: CH-CN
7 à 7,67 ppm: H aromatiques.

**Exemple 35: 1R trans 2,2-diméthyl 3-(3-bromo 1,2-propadiényl) cyclopropane carboxylate de méthyle.**

**Stade A: IR trans 2,2-diméthyl 3-(1RS hydroxy propynyl) cyclopropane carboxylate de méthyle.**

On refroidit à +10°C une solution de bromure d'éthynyl magnésium et coule en 15 minutes 12,5 g de IR trans 2,2-diméthyl 3-formyl cyclopropane carboxylate de méthyle dans 30 cm3 de tétrahydrofuranne. On agite 2 heures et demie à +10°C puis verse le mélange réactionnel sur une solution aqueuse d'acide chlorhydrique. On extrait à l'oxyde de diéthyle, concentre à sec les phases organiques sous pression réduite et récupère 15,17 g de produit que l'on purifie par chromatographie sur silice (éluant: benzène-acétate d'éthyle (7/3)). On obtient 11,74 g de produit attendu.

**Stade B: 1R trans 2,2-diméthyl 3-(3-bromo 1,2-propadiényl)**

**cyclopropane carboxylate de méthyle.**

On opère comme a l'exemple 27 stade B en utilisant 3,4 g du produit obtenu au stade A de l'exemple 35 et comme réactif 60 cm3 d'une solution de bromure de cuivre et de lithium dans le tétrahydrofuranne. Après chromatographie sur silice (éluant: hexane-acétate d'éthyle (9/1)), on obtient 1,5 g de produit attendu.

**Spectre de RMN**

1,2 et 1,29 ppm: $CH_3$ du cyclopropyle
3,72 ppm: $CO_2CH_3$
1,92 à 2,15 ppm et 6,07 à 6,18 ppm: chaîne allénique.

**Exemple 36: IR trans 2,2-diméthyl 3-(4-éthoxy 4-oxo 1,2-butadiényl) cyclopronane carboxylate de 1-(3-phénoxyphényl) méthyle (isomère A et isomère B).**

On opère comme à l'exemple 22 en utilisant l'acide préparé à l'exemple 21 et l'alcool(3-phénoxyphenyl) méthylique. On isole le produit attendu sous forme de 2 isomères. Isomère A: $/\alpha/_D = +107° + 2,5°$ (c = 0,8% $CHCl_3$) Isomère B: $/\alpha/_D = +108,5° + 3°$ (c = 0,5% $CHCl_3$).

**Exemples de compositions.**

**Exemple A: préparation d'un concentré soluble.**

- produit de l'exemple 2............................ 0,25 g
- Butoxyde de pipéronyle............................ 1 g
- Twéen 80......................................... 0,25 g
- Topanol A......................................... 0,1 g
- Eau............................................... 98,4 g

**Exemple B: préparation d'un concentré émulsifiable.**

- Produit de l'exemple 2............................ 0,015 g
- Butoxyde de pipéronyle............................ 0,5 g
- Topanol A......................................... 0,1 g
- Xylène............................................ 95,885 g
- Twéen 80.......................................... 3,5 g

**Exemple C: preparation d'un concentré émulsifiable.**

On effectue un mélange homogène de:
- produit de l'exemple 5............................ 1,5 g
- Twéen 80........................................... 20 g
- Topanol A.......................................... 0,1 g
- Xylène............................................. 78,4 g

**Exemple D: préparation d'une composition fumigène.**

On mélange d'une façon homogène:
- Produit de l'exemple 2.......................... 0,25 g
- poudre de tabu................................. 25 g
- poudre de feuille de cèdre.................. 40 g
- poudre de bois de pin....................... 33,75 g
- Vert brillant................................ 0,5 g
- Nitrophénol...................................... 0,5 g

**Etude de l'effet d'abattage des produits de l'invention sur mouche domestique**

Les insectes tests sont des mouches domestiques femelles âgées de 4 jours. On opère par pulvérisation directe à la concentration de 0,25 g/l en chambre de Kearns et March en utilisant comme solvant un mélange d'acétone (5%) et d'Isopar L (solvant pétrolier) (quantite de solvant utilisée: 2 ml en une seconde). On utilise 50 insectes par traitement. On effectue les contrôles toutes les minutes jusqu'à 10 minutes, puis à 15 minutes et l'on détermine le KT 50 par les méthodes habituelles.

Les resultats experimentaux obtenus sont résumes dans le tableau suivant:

| Produit de l'exemple | KT50 en mn |
|---|---|
| 1 | 3,99 |
| 2 | 2,81 |
| 3 | 4,05 |
| 4 | 3,65 |
| 5 | 3,22 |
| 6 | 4,54 |

**Revendications:**

1.- Sous toutes les formes isomères possibles, les composés de formule générale (I):

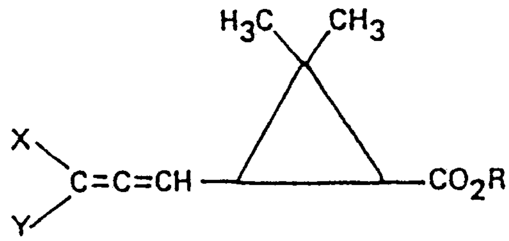

dans laquelle R représente le reste d'un alcool utilisé dans la synthèse des pyréthrinoides et

ou bien X et Y représentent chacun un atome d'hydrogène,

ou bien X représente un atome d'hydrogène ou un atome de chlore ou de brome et Y un radical alcoyle renfermant de 1 a 18 atomes de carbone ou un atome d'halogène, ou bien X représente un atome d'hydrogène ou d'halogène, ou un radical alcoyle renfermant de 1 à 18 atomes de carbone, et Y représente un radical $CO_2R'$, R' représentant un radical alcoyle renfermant de 1 à 18 atomes de carbone,

2.- Les composés de formule (I) tels que définis à la revendication 1 pour lesquels X et Y représentent chacun un atome d'hydrogène.

3.- Les composés de formule (I) selon la revendication 1 ou 2, pour lesquels R représente

- soit un radical alcoyle renfermant de 1 à 18 atomes de carbone,
- soit un radical benzyle eventuellement substitué par un ou plusieurs radicaux choisis dans le groupe constitué par les radicaux alcoyles comportant de 1 à 4 atomes de carbone, les radicaux alcényles comportant de 2 à 6 atomes de carbone, les radicaux alcényloxy comportant de 2 à 6 atomes de carbone, les radicaux alcadiényles comportant de 2 à 8 atomes de carbone, le radiaux méthylène dioxy et les atomes d'halogène,
- soit un groupement

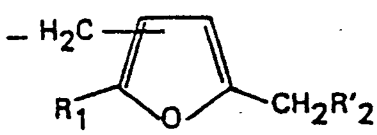

dans lequel le substituant $R_1$ represente un atome d'hydrogène ou un radi-cal méthyle et le substituant $R'_2$ un radical aryle monocyclique ou un groupement -C≡CH et notamment un groupement 5-benzyl 3-furyl méthyle,

- soit un groupement

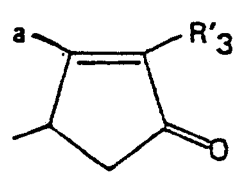

dans lequel a représente un atome d'hydrogène ou un radical methyle et $R'_3$ represente un radical organique aliphatique comportant 2 à 6 atomes de carbone et une ou plusieurs insaturations carbone-carbone et notamment le radical $-CH_2-CH=CH_2$, $-CH_2:CH=CH-CH_3$, $-CH_2-CH=CH-CH=CH_2$, $-CH_2-CH=CH-CH_2-CR_3$, $-CH_2-C{=}CH$ - soit un groupement

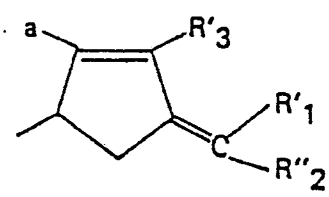

dans lequel a et $R_3$ conservent la même signification que précédemment, $R'_1$ et $R''_2$ identiques ou différents, représentent un atome d'hydrogène, un atome d'halogène, un radical alcoyle renfermant de 1 à 6 atomes de carbone, un radical aryle comportant de 6 à 10 atomes de carbone, un groupement alcoyloxycarbonyle comportant de 2 à 5 atomes de carbone, ou un groupement cyano,

soit un groupement

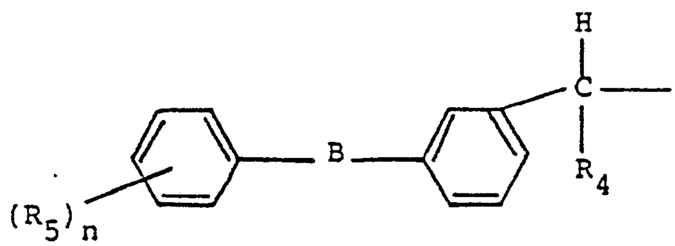

dans lequel B représente un atome d'oxygène ou de soufre, un groupement

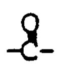

- ou $-CH_2-$ et $R_4$ represente un atome d'hydrogène,un radical -C≡N ou un - radical méthyle,un radical $-CO-NH_2$, un radical $-CSNH_2$ ou un radical -C≡CH, $R_5$ représente un atome d'halcgène ou un radical méthyle etn représente un nombre égal à 0, ou 2, et notamment le groupement 3-phénoxy benzyle, α-cyano 3-phénoxy benzyle,α -éthynyl 3-phénoxy benzyle, 3-benzoyl benzyle, 1-(3-phénoxyphényl) éthyle ou α-thioamido 3-phénoxy benzyle,

-soit un groupement

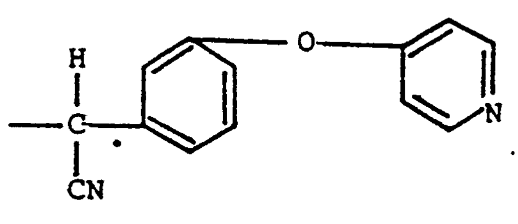

: soit un groupement

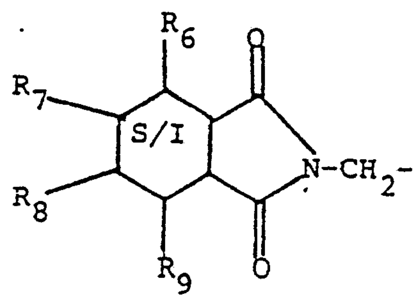

dans lequel les substituants $R_6$, $R_7$, $R_8$, $R_9$ representent un atome d'hydrogène, un atome de chlore ou un radical méthyle et dans lequel S/I symbolise un cycle aromatique ou un cycle analogue dihydro, tétrahydro ou hexahydro,

-soit un groupement (succinimido ou maléimido)-$CH_2$-,

- soit un groupement

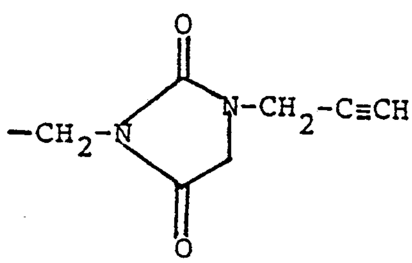

- soit un groupement

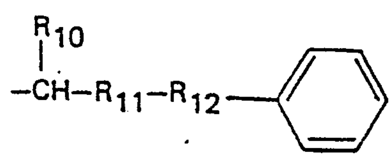

dans lequel $p_{10}$ représente un atome d'hydrogène ou un radical CN, $R_{12}$ représente un radical -$CH_2$- ou un atome d'oxygène, $R_{11}$ représente un radical thiazolyle ou thiadiazolyle dont la liaison avec 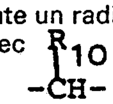 peut se trouver à l'une quelconque des positions disponibles, $R_{12}$ étant lie à $R_{11}$ par l'atome de carbone compris entre l'atome de soufre et un atome d'azote,

- soit un groupement

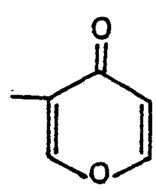

- soit un groupement

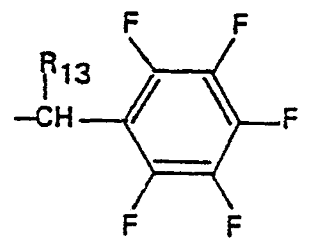

dans lequel $R_{13}$ represente un atome d'hydrogène ou un radical CN,
- soit un groupement

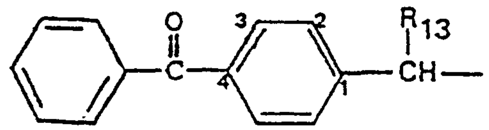

dans lequel $R_{13}$ est défini comme ci-dessus,
- soit un groupement

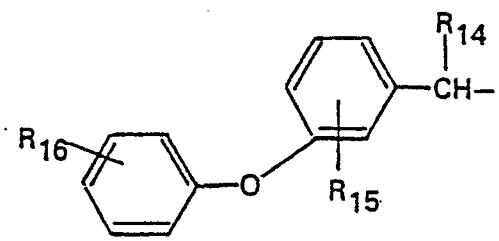

dans lequel $R_{14}$ represente un atome d'hydrogène, un radical méthyle, ethynyle ou cyano et $R_{15}$ et $R_{16}$, identiques ou différents, représentent un atome d'hydrogène, de fluor ou de brome, - soit un groupement

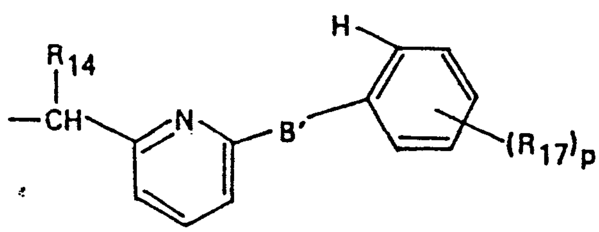

dans lequel $R_{14}$ est défini comme ci-dessus, chacun des $R_{17}$ représente independamment un groupement alcoyle renfermant de 1 à 4 atomes de carbone, alcoxy renfermant de 1 à 4 atomes de carbone, alcoylthio renfermant de 7 à 4 atomes de carbone, alcoyl sulfonyl renfermant de 1 à 4 atomes de carbone, trifluorométhyl,

3,4-methylène dioxy, chloro, fluoro ou bromo, p représente un nombre égal à 0,7 ou 2 et B' représente un atome d'oxygène ou un atome de soufre. 4°) Les composés de formule générale I, selon l'une quelconque des revendications 7 à 3, caractérisés en ce que le groupement R représente

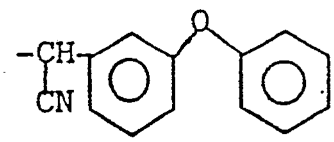

5°) Les composés de formule générale I, selon l'une quelconque des revendications 1 à 3, caractérisés en ce que le groupement R représente

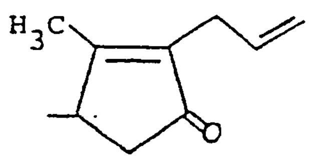

6°) Les composés de formule générale I, selon l'une quelconque des revendications 1 à 3, caractérisés en ce que le groupement R représente

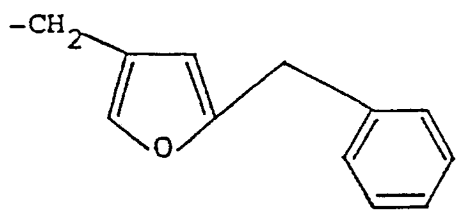

7°) Les composés de formule générale I selon l'une quelconque des revendications 1 à 3, caractérisés en ce que le groupement R représente

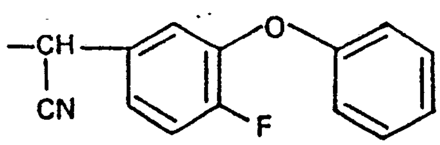

8°) Un procédé de préparation des composés de formule générale I selon l'une quelconque des revendicatios 1à 7, caractérisé en ce que l'on fait réagir un acide de formule générale (II)

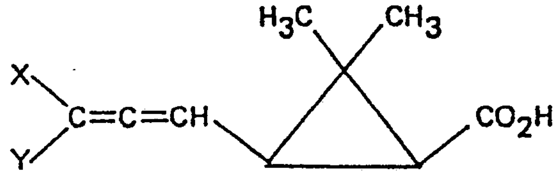
(II)

formule dans laquelle X et Y conservent les significations de la revendication 1 ou un dérivé fonctionnel de cet aci-de avec un alcool R-OH,Rconservant la même signification que dans la revendication 1, ou avec un dérivé fonctionnel de cet alcool. 9°) Un procédé de préparation des composés de formule générale I selon la revendication 8, caractérisé en ce que l'estérification de l'acide par l'alcool est effectuée en présence de

dicyclohexylcarbodiimide et de 4-dimethyl amino pyridine. 10°) procédé selon la revendication 8 pour la préparation de produits de formule I dans lesquels X=Y=H, caractérisé en ce que l'on prépare l'acide IR trans ou IR cis 2,2-dimethyl 3(1,2-propadiényl) cyclopropane carboxylique (produit de formule II dans lequel X=Y=H) en faisant réagir le butyl lithium sur un ester d'alcoyle de l'acide IR trans ou IR cis 2,2-diméthyl-3-(2,2-dibromovinyl) cyclopropane carboxylique, alcoyle représentant un alcoyle de 1 à 8 atomes de carbone, puis en faisant réagir le IR trans ou IR cis 2,2-diméthyl 3-éthynyl cyclopropane carboxylate d'alcoyle résultant-avec de la diisopropylamine, du formaldéhyde et du bromure cuivreux, puis en soumettant le 1R, trans ou le 1R, cis 2,2-diméthyl 3-(1,2-propadiényl) cyclopropane carboxylate d'alcoyle formé, à l'action d'un agent basique en présence d'eau. 11°) Application des composés de formule I tels que définis à l'une quelconque des revendications 1 à 7, à la lutte contre les parasites des végétaux, les parasites des locaux et les parasites des animaux à sang chaud. 12°) Les compositions destinées à la lutte contre les parasites des vegetaux, les parasites des locaux et les parasites des animaux à sang chaud, caractérisées en ce qu'elles renferment comme principe actif au moins un des produits definis a liune quelconque des revendications 1 à 7.

13°) Les compositions insectides renfermant comme principe actif au moins l'un des produits définis à l'une quelconque des revendications 1 à 7.

14°) Les compositions acaricides renfermant comme principe actif au moins l'un des produits définis à l'une quelconque des revendications 1 à 7.

15°) Les compositions nématicides renfermant comme principe actif au moins l'un des produits definis à l'une quelconque des

16°) Les compositions acaricides renfermant comme principe actif l'un au moins des produits définis à l'une quelconque des revendications 1 à 7, caractérisées en ce qu'elles sont utilisées dans la lutte contre les parasites des animaux à sang chaud, notamment contre les tiques et les gales.

17°) Les compositions destinées à l'alimentation animale renfermant commeprincipe actif au moins l'un des produits définis à l'une quelconque des revendications 1 à 7.

18°) Associations douées d'activite insecticide, acaricide ou nématicide, caractérisées en ce qu'elles contiennent comme matière active, d'une part un au moins des composés de formule générale (I) et d'autre part, un au moins des esters pyréthrinoïdes choisis dans le groupe constitué par les esters drallethrolones, d'alcool 3,4,5,6-tétrahydrophtalimido méthylique, d'alcool 5-benzyl 3-furyl méthylique, d'alcool 3-phénoxy benzylique et d'alcools α-cyano 3-phénoxy benzyliques des acides chrysanthémiques, par les esters d'alcool 5-benzyl 3-furyl méthylique des acides 2,2-diméthyl 3-(2-oxo 3-tétrahydrothio-phénylidène methyl) cyclopropane-7-carboxyliques, par les esters d'alcool 3-phénoxy benzylique et d'alcools α-cyano-3-phénoxy benzyliques des acides 2,2-diméthyl 3-(2,2-dichlorovinyl) cyclopropane-1-carboxyliques, par les esters d'alcools α-cyano 3-phénoxy benzyliques d'acides 2,2-diméthyl 3-(2,2-dibromovinyl) cyclopropane-7-carboxyliques, par les esters d'alcool 3-phénoxy benzylique des acides 2-parachlorophényl isopropyl acetiques, par les esters d'alléthrolones, d'alcool 3,4,5,6-tétrahydroph-talimido méthylique, d'alcool 5-benzyl 3-furyl méthylique, d'alcool 3-phenoxy benzylique et d'alcools α-cyano 3-phénoxy benzyliques des acides 2,2-diméthyl 3-1,2-2,2-tétrahaloéthyl cyclopropane-1-carboxyliques, dans lesquels "halo" représente un atome de fluor, de chlore ou de brome, étant entendu que les copules acides et alcools des esters pyréthrinoïdes ci-dessus, peuvent exister sous toutes leurs formes stereoisomères possibles.

19°) Les compositions selon l'une quelconque des revendications 12 à 18, caractérisées en ce qu'elles renferment en outre un synergiste des pyréthrinoîdes.

20°) A titre de produits chimiques nouveaux nécessaires à la mise en oeuvre du procédé de préparation des composésde l'invention tel que défini à la revendicatíon 8, les composés de formule II et notamment le compose de formule II pour lequel X=Y=H

**Patentansprüche**

1. In sämtlichen ihrer möglichen isomeren Formen die Verbindungen der allgemeinen Formel (I)

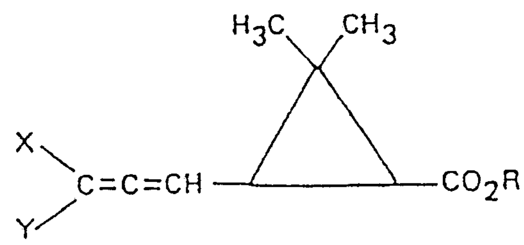

worin R den Rest eines bei der Synthese der Pyrethrinoide verwendeten Alkohols bedeutet; entweder X und Y jeweils ein Wasserstoffatom bedeuten oder M ein Wasserstoffatom oder ein Chlor- oder Bromatom bedeutet und Y einen Alkylrest mit 1 bis 18 Kohlenstoffatomen oder ein Halogenatom darstellt; oder X ein Wasserstoff- oder Halogenatom oder einen Alkylrest mit 1 bis 18 Kohlenstoffatomen bedeutet, und Y einen Rest $CO_2$ R' darstellt, worin R' einen Alkylrest mit 1 bis 18 Kohlenstoffatomen bedeutet.

2. Die Verbindungen der Formel (I) gemäß Anspruch 1, worin X und Y jeweils ein Wasserstoffatom bedeuten.

3. Die Verbindungen der Formel (I) gemäß Anspruch 1 oder 2, worin R bedeutet: entweder einen Alkylrest mit 1 bis 18 Kohlenstoffatomen oder einen Benzylrest, der gegebenenfalls substituiert ist durch einen oder mehrere Reste, ausgewählt unter den Alkylresten mit 1 bis 4 Kohlenstoffatomen, den Alkenylresten mit 2 bis 6 Kohlenstoffatomen, den Alkenyloxyresten mit 2 bis 6 Kohlenstoffatomen, den Alkadienylresten mit 4 bis 8 Kohlenstoffatomen, dem Methylendioxyrest und den Halogenatomen oder eine Gruppe

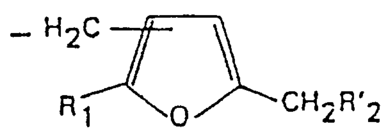

worin $R_1$ ein Wasserstoffatom oder einen Methylrest bedeutet und der Substituent $R_2$ einen monocyclischen Arylrest oder eine Gruppe $-C\equiv CH$ bedeutet und insbesondere eine 5-Benzyl-3-furyl-methyl-Gruppe oder eine Gruppe

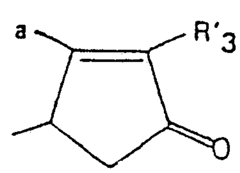

worin a ein Wasserstoffatom oder einen Methylrest bedeutet und $R'_3$ einen aliphatischen organischen Rest mit 2 bis 6 Kohlenstoffatomen und einer oder mehreren Kohlenstoff-Kohlenstoff-Ünsättigungen und insbesondere den Rest $-CH_2-CH=CH_2$, $-CH_2-CH=CH-CH_3$, $-CH_2-CH=CH-CH=CH_2$, $-CHCH=CH-CH_2-CH_3$ $-CH_2-C\equiv CH$ bedeutet oder eine Gruppe

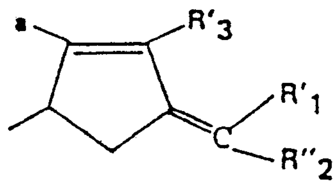

worin a und $R'_3$ die vorstehend angegebene Bedeutung besitzen, $R'_1$ und $R''_2$, die gleich oder verschieden sind, ein Wasserstoffatom, ein Halogenatom, einen Alkylrest mit 1 bis 6 Kohlenstoffatomen, einen Arylrest mit 6 bis 10 Kohlenstoffatomen, eine Alkoxycarbonylgruppe mit 2 bis 5 Kohlenstoffatomen oder eine Cyanogruppe bedeuten oder eine Gruppe

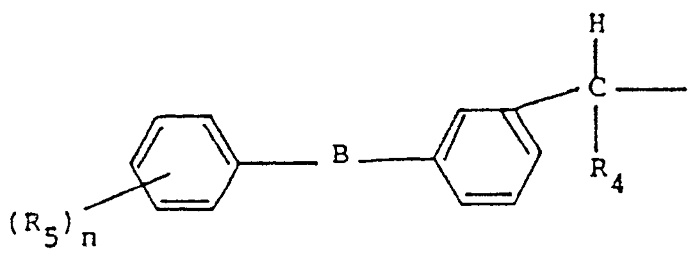

worin B ein Sauerstoff- oder Schwefelatom, eine Gruppe $-\overset{O}{\overset{\parallel}{C}}-$ oder $-CH_2-$ bedeutet und $R_4$ ein Wasserstoffatom, einen Rest $-C\equiv N$ oder einen Methylrest, einen Rest $-CO-NH_2$, einen Rest $-CSNH_2$ oder einen Rest $-C\equiv CH$ bedeutet, $R_5$ ein Halogenatom oder einen Methylrest darstellt und n eine Zahl entsprechend 0, 1 oder 2 bedeutet und insbesondere die 3-Phenoxybenzyl;α-Cyano-3-phenoxybenzyl-, α-Äthinyl-3-phenoxybenzyl-,3-Benzoylbenzyl-, 1-(3-Phenoxy-phenyl)-äthyl-oder α-Thioamido-3-phenoxybenzylgruppe, oder eine Gruppe

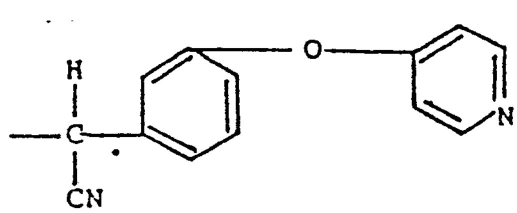

oder eine Gruppe

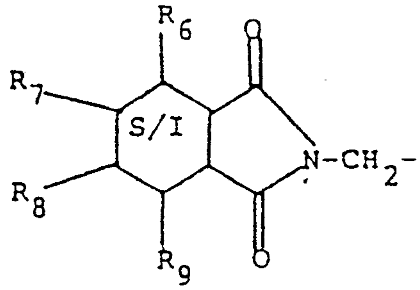

worin die Substituenten $R_6$, $R_7$, $R_8$ und $R_9$ ein Wasserstoffatom; ein Chloratom oder einen Methylrest darstellen und worin S/I einen aromatischen Ring oder einen analogen Dihydro-, Tetrahydro- oder Hexahydroring anzeigt, oder eine Gruppe (Succinimido oder Maleimido)-$CH_2$-, oder eine Gruppe

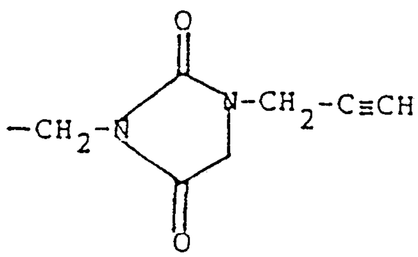

oder eine Gruppe

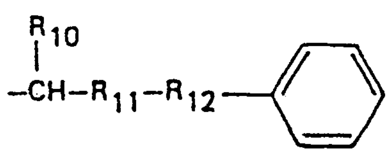

worin $R_{10}$ ein Wasserstoffatom oder einen CN-Rest bedeutet, $R_{12}$ einen Rest -$CH_2$- oder ein Sauerstoffatom darstellt, $R_{11}$ einen Thiazolyl- oder Thiadiazolylrest bedeutet, dessen Verknüpfung mit 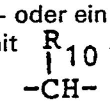 sich in irgendeiner der verfügbaren Positionen befinden kann, wobei $R_{12}$ an $R_{11}$ über das zwischen dem Schwefel- und einem Sauerstoffatom gelegene Kohlenstoffatom gebunden ist,
oder eine Gruppe

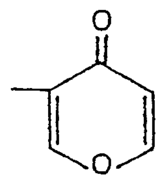

oder eine Gruppe

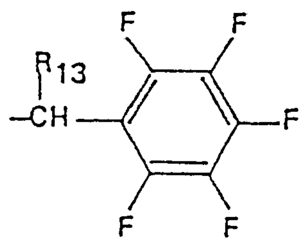

worin $R_{13}$ ein Wasserstoffatom oder einen CN-Rest bedeutet
oder eine Gruppe

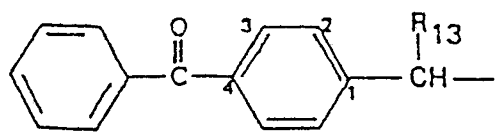

worin $R_{13}$ wie vorstehend definiert ist,
oder eine Gruppe

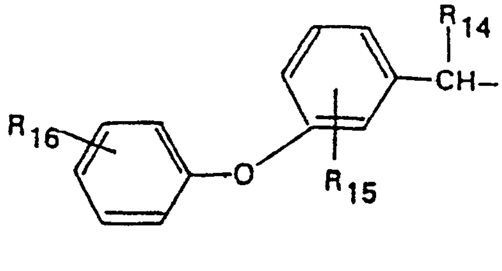

worin $R_{14}$ ein Wasserstoffatom, einen Methyl-, Äthinyloder Cyanorest bedeutet und $R_{15}$ und $R_{16}$, die gleich oder verschieden sind, ein Wasserstoff-,Fluor- oder Bromatom bedeuten,
oder eine Gruppe

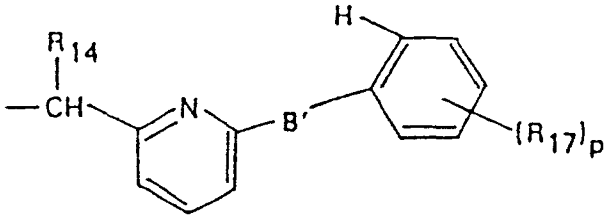

worin $R_{14}$ wie vorstehend definiert ist, ein jeder der Reste $R_{17}$ unabhängig eine Alkylgruppe mit 1 bis 4 Kohlenstoffatomen, eine Alkoxygruppe mit 1 bis 4 Kohlenstoffatomen, eine Alkylthiogruppe mit 1 bis 4 Kohlenstoffatomen, eine Alkylsulfonylgruppe mit 1 bis 4 Kohlenstoffatomen, Trifluormethyl, 3,4-Methylendioxy, ein Chlor-, Fluor- oder Bromatom bedeutet, p eine Zahl entsprechend 0, 1 oder 2 bedeutet und B' ein Sauerstoff- oder Schwefelatom darstellt.

4. Die Verbindungen der allgemeinen Formel (I) gemäß einem der Ansprüche 1 bis 3, dadurch gekennzeichnet, daß die Gruppe R

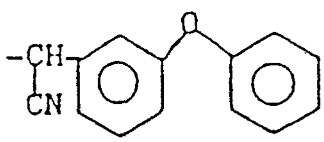

bedeutet.

5.Die Verbindungen der allgemeinen Formel (I) gemäß einem der Ansprüche 1 bis 3, dadurch gekennzeichnet, daß die Gruppe R

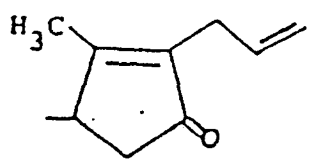

bedeutet.

6.Die Verbindungen der allgemeinen Formel (I) gemäß einem der Ansprüche 1 bis 3, dadurch gekennzeichnet, daß die Gruppe R

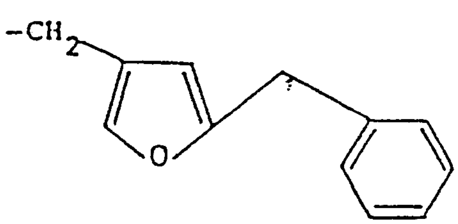

bedeutet.

7.Die Verbindungen der allgemeinen Formel (I) gemäß einem der Ansprüche 1 bis 3, dadurch gekennzeichnet,

daß die Gruppe R

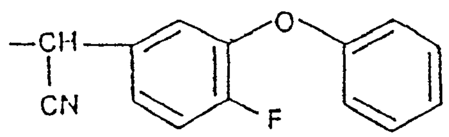

bedeutet.

8.Verfahren zur Herstellung der Verbindungen der allgemeinen Formel (I) gemäß einem der Ansprüche 1 bis 7 dadurch gekennzeichnet, daß man eine Säure der allgemeinen Formel (II)

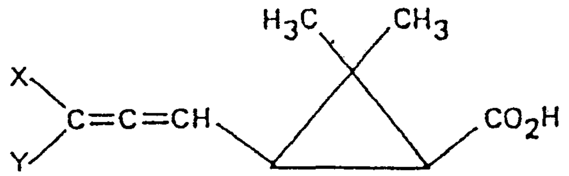

(II)

worin X und Y, die in Anspruch 1 angegebenen Bedeutungen besitzen,oder ein funktionelles Derivat dieser Säure mit einem Alkohol R-OH, worin R die in Anspruch 1 angegebene Bedeutung besitzt,oder mit einem funktionellen Derivat dieses Alkohols umsetzt.

9.Verfahren zur Herstellung der Verbindungen der allgemeinen Formel (I) gemäß Anspruch 8, dadurch gekennzeichnet, daß man die Veresterung der Säure mit dem Alkohol in Gegenwart von Dicyclohexylcarbodiimid und 4-Dimethylaminopyridin durchführt.

10.Verfahren gemäß Anspruch 8 zur Herstellung der Produkte der Formel (I), worin X=Y=H, dadurch gekennzeichnet, daß man die IR,trans- oder 1R,cis-2,2-Dimethyl-3-(1,2-propa-dienyl)-cyclopropan-carbonsäure (Produkt der Formel (II), worin X=Y=H) herstellt, indem man Butylithium mit einem Alkylester der 1R,trans- oder 1R,cis-2,2-Dimethyl-3-(2,2-dibromvinyl)-cyclopropan-carbonsäure umsetzt, wobei Alkyl einen Alkylrest mit 1 bis 8 Kohlenstoffatomen bedeutet, danach das entstandene Alkyl(1R,trans- oder 1R,cis-2,2-Di-methyl-3-äthinyl-cyclopropan-carboxylat) mit Diisopropylamin, Formaldehyd, Kupfer(I)-Bromid umsetzt und anschließend das gebildete Alkyl-[1R,trans- oder 1R,cis-2,2'Dimethy1-3-(1,2-propadienyl)-cyclopropan-carboxylat] der Einwirkung eines basischen Mittels in Gegenwart von Wasser unterzieht.

11. Verwendung der Verbindungen der Formel (I) gemäß einem der Ansprüche 1 bis 7 bei der Bekämpfung von Parasiten der Pflanzen, Parasiten von Räumlichkeiten und Parasiten warmblütiger Tiere.

12. Zusammensetzungen für die Bekämpfung von Parasiten der Pflanzen, Parasiten von Räumlichkeiten und Parasiten warmblütiger Tiere, dadurch gekennzeichnet, daß sie als Wirkstoff zumindest eines der Produkte gemäß einem der Ansprüche 1 bis 7 enthalten.

13. Insektizide Zusammensetzungen,enthaltend als Wirkstoff zumindest eines der Produkte gemäß einem der Ansprüche 1 bis 7.

14. Akarizide Zusammensetzungen,enthaltend als Wirkstoff zumindest eines der Produkte gemäß einem der Ansprüche 1 bis 7.

15. Nematizide Zusammensetzungen,enthaltend als Wirkstoff zumindest eines der Produkte gemäß einem der Ansprüche

16. Akarizide Zusammensetzungen, enthaltend als Wirkstoff zumindest eines der Produkte gemäß einem der Ansprüche 1 bis 7, dadurch gekennzeichnet, daß sie bei der Bekämpfung von Parasiten warmblütiger Tiere, insbesondere bei der Bekämpfung von Zecken und Krätzmilben eingesetzt werden.

17. Zusammensetzungen für die tierische Ernährung, enthaltend als Wirkstoff zumindest eines der Produkte gemäß einem der Ansprüche 1 bis 7.

18. Mit insektizider, akarizider oder nematizider Aktivität versehene Assoziationen, dadurch gekennzeichnet, daß sie als Wirkstoff einesteils zumindest eine der Verbindungen der allgemeinen Formel (I) und anderenteils zumindest einen der Pyrethrinoidester, ausgewählt unter den Estern der Allethrolone, des 3,4,5,6 Tetrahydrophthalimidomethylalkohols, des 5-Benzyl-3-furyl-methylalkohols, des 3-Phenoxybenzylalkohols und derα-Cyano-3-phenoxybenzyl-alkohole der Chrysanthemumsäuren, unter den Estern des 5-Benzyl-3-furyl-methylalkohols der 2,2-Dimethyl-3-(2-oxo-3-tetrahydrothiophenylidenmethyl)-cyclopropan-1-carbonsäuren, unter den Estern des 3-Phenoxy-benzylalkohols und der α-Cyano-3-phenoxybenzylalkohole der 2,2-Dimethyl-3-(2,2-di-chlorvinyl)-cyclopropan-1-carbonsäuren, unter den Estern der α-Cyano-3-phenoxybenzylalkohole der 2,2-Dimethyl-3-(2,2-dibromvinyl)-cyclopropan-1-carbonsäuren, unter den Estern des 3-Phenoxybenzylalkohols der 2-p-Chlorphenyl-isopropylessigsäuren, unter den Estern der Allethrolone, des 3,4,5,6-Tetrahydrophthalimidomethylalkohols, des 5-Benzyl-3-furyl-methylalkohols, des 3-Phenoxybenzylalkohols und der α-Cyano-3-phenoxybenzylalkohole der 2,2-Dimethyl-3-(1,2,2,2-tetra-haloäthyl)-cyclopropan-1-carbonsäuren enthalten, worin "halo" ein Fluor-, Chlor- oder Bromatom bedeutet,wobei die Säure- und Alkoholverknüpfungskomponenten der vorstehenden Pyrethrinoidester in sämtlichen ihrer möglichen stereoisomeren Formen vorliegen können.

19. Zusammensetzungen gemäß einem der Ansprüche 12 bis 18, dadurch gekennzeichnet, daß sie außerdem einen Synergisten der Pyrethrinoide enthalten.

20. Als neue chemische Produkte, die für die Durchführung des Verfahrens zur Herstellung der erfindungsgemäßen Verbindungen gemäß Anspruch 8 erforderlich sind, die Verbindungen der Formel (II) und insbesondere die Verbindung der Formel (II), worin X=Y=H.

## Claims

1. - In all their possible isomeric forms, the compounds with the general formula (I):

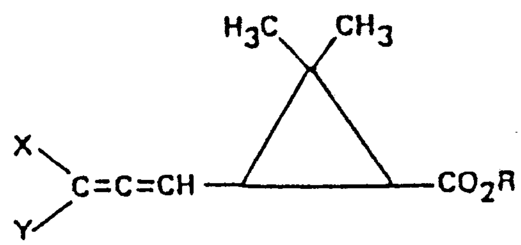

in which R represents the residue of an alcohol used in the synthesis of the pyrethinoids and

either X and Y each represent a hydrogen atom

or X represents a hydrogen atom or a chlorine or bromine atom and Y represents an alkyl radical containing from 1 to 18 carbon atoms or a halogen atom,

or X represents a hydrogen or halogen atom, or an alkyl radical containing from 1 to 18 carbon atoms, and Y represents a radical $CO_2R'$, R' representing an alkyl radical containing from 1 to 18 carbon atoms.

2. - The compounds with the formula (I) as defined in Claim 1 for which X and Y each represent a hydrogen atom.

3. The compounds with the formula (I) according to Claim 1 or 2, for which R represents

- either an alkyl radical containing from 1 to 18 carbon atoms,
- or a benzyl radical, possibly substituted by one or more radicals chosen from the group constituted by the alkyl radicals containing from 1 to 4 carbon atoms, the alkenyl radicals containing from 2 to 6 carbon atoms, the alkenyloxy radicals containing from 2 to 6 carbon atoms, the alkadienyl radicals containing from 4 to 8 carbon atoms, the methylene dioxy radical and the halogen atoms,
- or a group

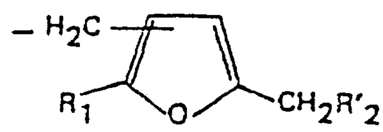

in which the substituent $R_1$ represents a hydrogen atom or a methyl radical and the substituent $R'_2$ represents a monocyclic aryl radical or a group -C≡CH and in particular a 5-benzyl-3-furyl methyl group,

- or a group

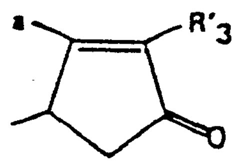

in which a represents a hydrogen atom or a methyl radical and $R'_3$ represents an aliphatic organic radical containing 2 to 6 carbon atoms and one or more carbon-carbon unsaturations and in particular the radical $-CH_2-CH=CH_2$, $-CH_2-CH=CH-CH_3$, $-CH_2-CH=CH-CH=CH_2$, $-CH_2-CH=CH-CH_2-CH_3$, $-CH_2-C\equiv CH$,

- or a group

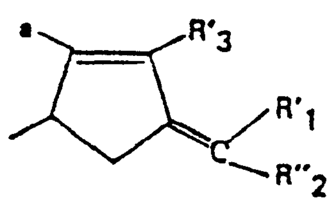

in which a and $R'_3$ retain the same significance as before, $R'_1$ and $R''_2$ being identical or different, represent a hydrogen atom, a halogen atom, an alkyl radical containing from 1 to 6 carbon atoms, an aryl radical containing from 6 to 10 carbon atoms, an alkyloxycarbonyl group containing from 2 to 5 carbon atoms, or a cyano group,

- or a group

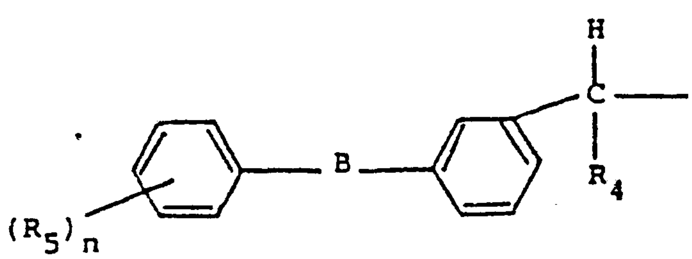

in which B represents an oxygen or sulphur atom, a group $-\overset{O}{\overset{\|}{C}}-$ or $-CH_2-$ and $R_4$ represents a hydrogen atom, a radical -C=N or a methyl radical, a radical $-CO-NH_2$, a radical $-CSNH_2$ or a radical $-C\equiv CH$, $R_5$ represents a

halogen atom or a methyl radical and n represents a numeral 0, 1 or 2, and in particular the group 3-phenoxybenzyl, α-cyano-3-phenoxybenzyl, α -ethynyl-3-phenoxybenzyl, 3-benzoylbenzyl, 1-(3-phenoxyphenyl)ethyl or α -thioamido-3-phenoxybenzyl, - or a group

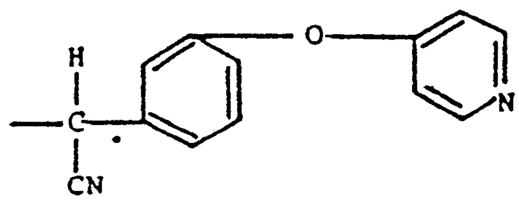

- or a group

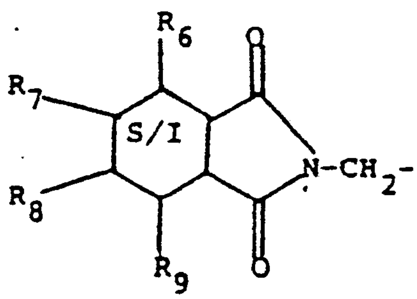

in which the substituents $R_6$, $R_7$, $R_8$, $R_9$ represent a hydrogen atom, a chlorine atom or a methyl radical, and in which S/I symbolizes an aromatic ring or a similar dihydro, tetrahydro or hexahydro ring,
- or a (succinimido or maleimido)-$CH_2$- group, - or a group

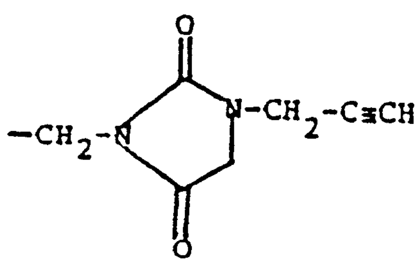

- or a group

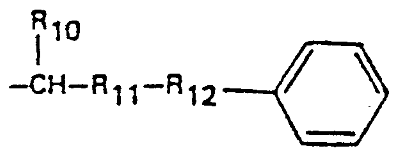

in which $R_{10}$ represents a hydrogen atom or a radical CN, $R_{12}$ represents a radical $-CH_2-$ or an oxygen atom, $R_{11}$ represents a thiazolyl or thiadiazolyl radical, of which the bond with

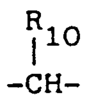

may be at any one of the available positions, $R_{12}$ being bonded to $R_{11}$ by the carbon atom included between the sulphur atom and a nitrogen atom,

- or a group

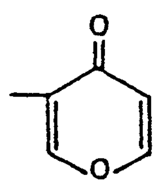

- or a group

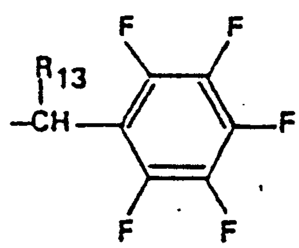

in which $R_{13}$ represents a hydrogen atom or a radical CN,

- or a group

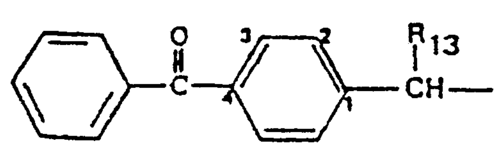

in which $R_{13}$ is defined as above,

- or a group

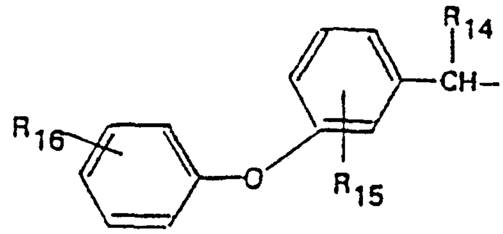

in which $R_{14}$ represents a hydrogen atom, a methyl, ethynyl or cyano radical and $R_{15}$ and $R_{16}$, being identical or different, represent a hydrogen, fluorine or bromine atom,

- or a group

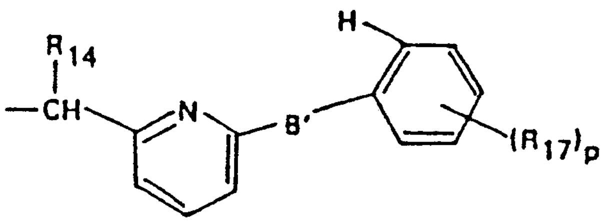

in which $R_{14}$ is defined as above, each of the $R_{17}$'s represents independently an alkyl group containing from 1 to 4 carbon atoms, an alkoxy group containing from 1 to 4 carbon atoms, an alkylthio group containing from 1 to 4 carbon atoms, an alkylsulphonyl group containing from 1 to 4 carbon atoms, a trifluoromethyl group, a 3,4-methylenedioxy group, or a chloro, fluoro or bromo group, p represents a numeral 0, 1 or 2 and B' represents an oxygen atom or a sulphur atom.

4.- The compounds with the general formula I, according to any one of the Claims 1 to 3, characterized in that the group R represents

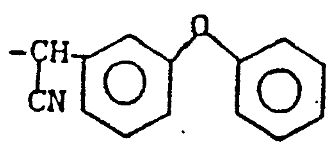

5.- The compounds with the general formula I, according to any one of the Claims 1 to 3, characterized in that the group R represents

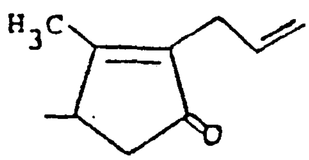

6.- The compounds with the general formula I, according to any one of the Claims 1 to 3, characterized in that the group R represents

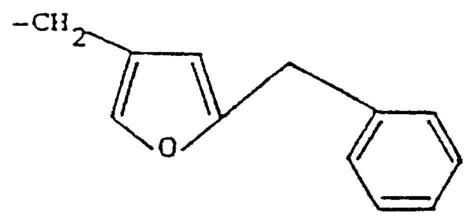

7.- The compounds with the general formula I according to any one of the Claims 1 to 3, characterized in that the group R represents

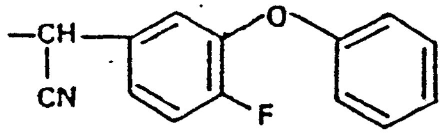

8.- A preparation process for the compounds with the general formula I according to any one of the Claims 1 to 7 characterized in that an acid with the general formula (II)

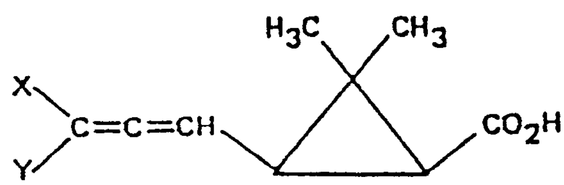

(II)

in which formula X and Y retain the significances of Claim 1, or a functional derivative of this acid is made to react with an alcohol R-OH, R retaining the same significance as in Claim 1, or with a functional derivative of this alcohol.

9.- A preparation process for the compounds with the general formula I according to Claim 8, characterized in that the esterification of the acid by the alcohol is carried out in the presence of dicyclohexylcarbodiimide and 4-dimethylamino pyridine.

10.- Process according to Claim 8 for the preparation of products with the formula I in which X=Y=H, characterized in that IR trans or IR cis 2,2-dimethyl-3-(1,2-propadienyl) cyclopropane carboxylic acid (product with the formula II in which X=Y=H) is prepared by making butyl-lithium react on an alkyl ester of IR trans or IR cis 2,2-dimethyl-3-(2,2-dibromovinyl)cyclopropane carboxylic acid, alkyl representing an alkyl with 1 to 8 carbon atoms, then by making the resulting IR trans or IR cis 2,2-dimethyl-3-ethynylcyclopropane carboxylate of alkyl react with diisopropylamine, formaldehyde and cuprous bromide, then by submitting the 1R trans or 1R cis 2,2-dimethyl-3-(1,2-propadienyl)cyclopropane carboxylate of alkyl formed to the action of a basic agent in the presence of water.

11.- Use of the compounds with the formula I as defined in any one of the Claims 1 to 7 to combat the parasites of vegetation, the parasites of premises and the parasites of warm-blooded animals.

12.- Compositions intended to combat the parasites of vegetation, the parasites of premises and the parasites of warm-blooded animals, characterized in that they contain as active principle at least one of the products defined in any one of the Claims 1 to 7.

13.- Insecticide compositions containing as active principle at least one of the products defined in any one of the Claims 1 to 7

14.- Acaricide compositions containing as active principle at least one of the products defined in any one of the Claims 1 to 7.

15.- Nematocide compositions containing as active principle at least one of the products defined in any one of the Claims 1 to 7.

16.- Acaracide compositions containing as active principle at least one of the products defined in any one of the Claims 1 to 7, characterized in that they are used to combat the parasites of warm-blooded animals, in

particular ticks and mites.

17.- Compositions intended as animal foodstuffs containing as active principle at least one of the products defined in any one of the Claims 1 to 7.

18.- Combinations endowed with insecticide, acaricide or nematocide activity, characterized in that they contain, as active material, for the one part at least one of the compounds with the general formula (I) and for the other part, at least one of the pyrethrinoid esters chosen from the group constituted by the esters of allethrolones, of 3,4,5,6-tetrahydrophthalimidomethyl alcohol, of 5-benzyl-3-furylmethyl alcohol, of 3-phenoxybenzyl alcohol and of α-cyano-3-phenoxybenzyl alcohols with chrysanthemic acids, by the esters of 5-benzyl-3-furylmethyl alcohol with 2,2-dimethyl-3-(2-oxo-3-tetrahydrothiophenylidenemethyl)-cyclopropane-1-carboxylic acids, by the esters of 3-phenoxy-benzyl alcohol and α -cyano-3-phenoxybenzyl alcohols with 2,2-dimethyl-3-(2,2-dichlorovinyl)cyclopropane-1-carboxylic acids, by the esters of α -cyano-3-phenoxybenzyl alcohols with 2,2-dimethyl-3-(2,2-dibromovinyl)cyclopropane-1-carboxylic acids, by the esters of 3-phenoxybenzyl alcohol with 2-parachlorophenylisopropyl acetic acids, by the esters of allethrolones, of 3,4,5,6-tetrahydrophthalimidomethyl alcohol, of 5-benzyl-3-furylmethyl alcohol, of 3-phenoxy-benzyl alcohol and of α -cyano-3-phenoxybenzyl alcohols with 2,2-dimethyl-3-(1,2,2,2-tetrahaloethyl)cyclopropane-1-carb-oxylic acids, in which "halo" represents a fluorine, chlorine or bromine atom, it being understood that the acid and alcohol copulas of the above pyrethrinoid esters can exist in all their possible stereo-isomeric forms.

19.- Compositions according to any one of the Claims 12 to 18, characterized in that they contain in addition, a synergist of the pyrethrinoids.

20.- As new chemical products necessary for putting into operation the preparation process for the invention compounds as defined in Claim 8, the compounds with the formula 11, and in particular, the compound with the formula II for which X=Y=H.